(19)

**Europäisches Patentamt**
**European Patent Office**
**Office européen des brevets**

(11) **EP 4 410 383 B1**

(12) **EUROPEAN PATENT SPECIFICATION**

(45) Date of publication and mention
of the grant of the patent:
**22.04.2026 Bulletin 2026/17**

(21) Application number: **23190895.5**

(22) Date of filing: **10.08.2023**

(51) International Patent Classification (IPC):
*A61K 8/73* (2006.01)     *A61Q 1/00* (2006.01)
*A61Q 19/00* (2006.01)     *C08B 3/06* (2006.01)
*C09D 103/02* (2006.01)     *C09D 105/00* (2006.01)
*C09D 105/08* (2006.01)     *C09D 179/02* (2006.01)

(52) Cooperative Patent Classification (CPC):
(C-Sets available)
**C08B 3/06; A61K 8/731; A61Q 1/00; A61Q 19/00;**
**C09D 103/02; C09D 105/00; C09D 105/08;**
**C09D 179/02**          (Cont.)

(54) **CELLULOSIC PARTICLE**

ZELLULOSEPARTIKEL

PARTICULE CELLULOSIQUE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **03.02.2023 JP 2023015493**

(43) Date of publication of application:
**07.08.2024 Bulletin 2024/32**

(73) Proprietor: **FUJIFILM Business Innovation Corp.**
**Minato-ku**
**Tokyo (JP)**

(72) Inventors:
• **YAO, Kenji**
  **Minamiashigara-shi, Kanagawa (JP)**
• **YOSHIKAWA, Hideaki**
  **Minamiashigara-shi, Kanagawa (JP)**
• **ISHIZUKA, Takahiro**
  **Minamiashigara-shi, Kanagawa (JP)**
• **MATOBA, Shota**
  **Minamiashigara-shi, Kanagawa (JP)**
• **NAITO, Ayu**
  **Minamiashigara-shi, Kanagawa (JP)**

• **KASHIWAGI, Satomi**
  **Minamiashigara-shi, Kanagawa (JP)**
• **YOSHIDA, Kazusei**
  **Minamiashigara-shi, Kanagawa (JP)**
• **IWADATE, Yuko**
  **Minamiashigara-shi, Kanagawa (JP)**
• **OKI, Masahiro**
  **Minamiashigara-shi, Kanagawa (JP)**
• **IWANAGA, Takeshi**
  **Minamiashigara-shi, Kanagawa (JP)**
• **TAGUCHI, Tetsuya**
  **Minamiashigara-shi, Kanagawa (JP)**
• **HAMANO, Hirokazu**
  **Minamiashigara-shi, Kanagawa (JP)**

(74) Representative: **Kurig, Thomas**
**Becker Kurig & Partner**
**Patentanwälte mbB**
**Bavariastraße 7**
**80336 München (DE)**

(56) References cited:
**EP-A1- 4 116 357**      **WO-A1-2022/137679**
**CN-A- 114 555 678**

**(Cont. next page)**

EP 4 410 383 B1

(52) Cooperative Patent Classification (CPC): (Cont.)

C-Sets
**C08L 1/02, C08K 5/175, C08K 5/101;**
**C08L 1/02, C08K 5/5415, C08K 5/101;**
**C08L 1/02, C08L 3/02, C08K 3/26, C08K 5/07,**
**C08K 5/101;**
**C08L 1/02, C08L 3/02, C08L 91/00, C08K 5/07,**
**C08K 5/101;**
**C08L 1/02, C08L 3/02, C08L 91/00, C08K 5/07,**
**C08K 5/101, C08K 3/36;**
**C08L 1/02, C08L 5/00, C08K 3/26, C08K 5/07,**
**C08K 5/101;**
**C08L 1/02, C08L 5/00, C08L 91/00, C08K 5/07,**
**C08K 5/101;**
**C08L 1/02, C08L 5/08, C08K 3/26, C08K 5/07,**
**C08K 5/101;**
**C08L 1/02, C08L 5/08, C08L 91/00, C08K 5/07,**
**C08K 5/101;**
**C08L 1/02, C08L 5/08, C08L 91/00, C08K 5/07,**
**C08K 5/101, C08K 3/36;**
**C08L 1/02, C08L 79/02, C08K 3/26, C08K 5/07,**
**C08K 5/101;**
**C08L 1/02, C08L 79/02, C08L 91/00, C08K 5/07,**
**C08K 5/101, C08K 3/36;**
**C08L 1/02, C08L 91/00, C08K 5/101;**
**C08L 1/12, C08L 1/286, C08K 3/26;**
**C08L 1/12, C08L 1/286, C08K 3/26, C08K 5/07;**
**C08L 1/12, C08L 1/286, C08K 3/26, C08K 5/07,**
**C08K 5/101;**
**C08L 1/12, C08L 1/286, C08K 3/26, C08K 5/101;**
**C08L 1/12, C08L 1/286, C08K 5/07, C08K 5/101;**
**C08L 1/14, C08L 1/286, C08K 3/26;**
**C08L 1/14, C08L 1/286, C08K 3/26, C08K 5/07;**
**C08L 1/14, C08L 1/286, C08K 3/26, C08K 5/101;**
**C08L 1/14, C08L 1/286, C08L 91/00, C08K 5/101**

# EP 4 410 383 B1

**Description**

Background

(i) Technical Field

The present disclosure relates to a cellulosic particle.

(ii) Related Art

In Japanese Unexamined Patent Application Publication No. 2022-099605, "resin beads obtained by surface treatment of core beads formed from a resin having cellulose as a main component by a solid surface treatment agent, in which the volume-based cumulative 50% particle size is 50 $\mu$m or less, the sphericity is 0.7-1.0, the surface smoothness is 70-100%, and the degree of crystallization is 60% or less." are proposed.

In Japanese Unexamined Patent Application Publication No. 2020-132616, "oily solid cosmetics containing surface-treated spherical cellulose powder with an average particle size of 1.0-30.0 $\mu$m." is proposed.

WO 2022/137679 A1 discloses resin beads formed of a resin containing cellulose as a main component, a method for producing the resin beads, and products such as cosmetics obtained by using the resin beads.

EP 4116357 A1 discloses resin beads containing a cellulose derivative as a main component, a method for producing the resin beads, and products, such as cosmetics, obtained using the resin beads.

CN 114555678 A discloses a cellulose particle and a method for producing a cellulose particle.

Summary

The present disclosure is provided by the appended set of claims.

Accordingly, it is an object of the present disclosure to provide a cellulosic particle as defined in the appended claim 1 that has a fluffy feel compared with if at least one of the amount of the hydrocarbon ester and the amount of the ketone in the cellulosic particle is less than 3 ppm or more than 1000 ppm.

It should be noted that the "fluffy feel" is a measure represented in "Fluffy Feel Evaluations" in the Examples below.

According to the appended claim 2 of the present disclosure, there is provided a cellulosic particle that has a fluffy feel compared with if the cellulosic particle has a coating layer containing a silane compound.

According to the appended claim 3 of the present disclosure, there is provided a cellulosic particle that has a fluffy feel compared with if the fatty acid is a fatty acid having fewer than 16 or more than 22 carbon atoms or if the number of carbon atoms in the fatty acid metallic salt is fewer than 16 or more than 22.

According to the appended claim 4 of the present disclosure, there is provided a cellulosic particle that has a fluffy feel compared with if the fatty acid is an unsaturated fatty acid or if the fatty acid metallic salt is an unsaturated fatty acid metallic salt.

According to the appended claim 5 of the present disclosure, there is provided a cellulosic particle that has a fluffy feel compared with if the cellulosic particle has an intermediate layer containing arginine or dextrin.

According to the appended claim 6 of the present disclosure, there is provided a cellulosic particle that has a fluffy feel compared with if the cellulosic particle has no inorganic particle as an external additive.

According to the appended claim 7 of the present disclosure, there is provided a cellulosic particle that has a fluffy feel compared with if the volume-average particle diameter is less than 3 $\mu$m or 10 $\mu$m or more.

According to the appended claim 8 of the present disclosure, there is provided a cellulosic particle that has a fluffy feel compared with if the upper geometric standard deviation by number GSDv is less than 1.0 or greater than 1.7.

According to the appended claim 9 of the present disclosure, there is provided a cellulosic particle that has a fluffy feel compared with if the sphericity is less than 0.7.

According to the appended claim 10 of the present disclosure, there is provided a cellulosic particle that has a fluffy feel compared with if the surface smoothness is less than 50%.

According to the appended claim 11 of the present disclosure, there is provided a cellulosic particle that has a fluffy feel compared with if the number-average molecular weight of the cellulose is less than 37000.

According to the appended claim 12 of the present disclosure, there is provided a cellulosic particle that has a fluffy feel compared with if the number-average molecular weight of the cellulose is less than 45000.

Detailed Description

Exemplary embodiments as examples of the present disclosure will now be described. These descriptions and the Examples are intended to illustrate exemplary embodiments and not intended to limit the scope of aspects of the present disclosure.

In a series of numerical ranges presented herein, an upper or lower limit specified in one numerical range may be substituted with the upper or lower limit of another numerical range in the same series.

A constituent may include multiple corresponding substances.

When the amount of a constituent in a composition is mentioned herein, and if multiple substances corresponding to the constituent are present in the composition, the mentioned amount represents the total amount of the multiple substances present in the composition unless stated otherwise.

"Step" refers not only to an independent step; even if a step cannot be clearly distinguished from another, the step is included in this term as long as its intended action is achieved.

Cellulosic Particles

Cellulosic particles according to an exemplary embodiment contain cellulose as their base constituent and at least one selected from the group consisting of a hydrocarbon ester and a ketone, and at least one of the amount of the hydrocarbon ester and the amount of the ketone is 3 ppm or more and 1000 ppm or less.

Configured as described above, the cellulosic particles according to this exemplary embodiment are cellulosic particles having a fluffy feel. Possible reasons are as follows.

Known cellulosic particles tend to be lacking in fluffy feel because hydrogen bonding inside and outside the cellulose as their base constituent is firm.

The cellulosic particles according to this exemplary embodiment contain cellulose as their base constituent and at least one selected from the group consisting of a hydrocarbon ester and a ketone. Hydrocarbon esters tend to be likely to penetrate between cellulose molecules and, at the same time, are likely to weaken hydrogen bonding inside and outside the cellulose molecules because hydroxyl groups contained in the cellulose and the ester group in the hydrocarbon ester interact. Ketones, too, are likely to weaken hydrogen bonding inside and outside the cellulose molecules because they interact with hydroxyl groups contained in the cellulose molecules. The cellulosic particles according to this exemplary embodiment, furthermore, are likely to have an improved fluffy feel because the higher-order structure of the cellulose is disturbed by virtue of the presence of at least one selected from the group consisting of a hydrocarbon ester and a ketone.

The amount of the hydrocarbon ester or ketone, furthermore, is 3 ppm or more and 1000 ppm or less. By setting the amount of the hydrocarbon ester or ketone to 3 ppm or more, it is easier for these compounds to penetrate into cellulose molecules. By setting the amount of the hydrocarbon ester or ketone to 1000 ppm or less, their exudation from the cellulosic particles is reduced.

For these reasons, presumably, the cellulosic particles according to this exemplary embodiment are cellulosic particles having a fluffy feel.

Constituents of the Cellulosic Particles

Cellulose

The cellulosic particles according to this exemplary embodiment contain cellulose as their base constituent.

In this context, containing cellulose as a base constituent means that the amount of cellulose relative to the cellulosic particles is 90% by mass or more.

When the cellulosic particles have the coating and intermediate layers described later herein, containing cellulose as a base constituent means that the cellulose content relative to the core particle is 90% by mass or more.

The number-average molecular weight of the cellulose may be 37000 or more, preferably 45000 or more.

There is no particular upper limit, but for example, the number-average molecular weight of the cellulose may be 100000 or less.

By setting the number-average molecular weight of the cellulose to 37000 or more, it is likely that the number of hydroxyl groups exposed on the surface of the cellulosic particles is small. It is, therefore, likely that dispersibility in a dispersion is improved. By setting the number-average molecular weight of the cellulose to 45000 or more, it is more likely that the number of hydroxyl groups exposed on the surface of the cellulosic particles is small. It is, therefore, more likely that dispersibility in a dispersion is improved.

The number-average molecular weight of the cellulose is measured by gel permeation chromatography (differential refractometer, Optilab T-rEX, Wyatt Technology; multiangle light scattering detector, DAWN HELEOS II, Wyatt Technology; columns, one TSKgel $\alpha$-M and one $\alpha$-3000, Tosoh) with dimethylacetamide (with the addition of 0.1 M lithium chloride) as the eluent.

Hydrocarbon Ester

A hydrocarbon ester is a compound having at least one ester group and in which only a hydrocarbon group binds to the ester group.

The number of ester groups that the hydrocarbon ester contains may be one or two, preferably one.

The number of carbon atoms that the hydrocarbon ester contains may be one or more and eight or fewer, preferably one or more and five or fewer, more preferably one or more and three or fewer.

The hydrocarbon group that the hydrocarbon ester contains may be a saturated hydrocarbon group, and examples include the methyl, ethyl, n-propyl, isopropyl, n-butyl, isobutyl, pentyl, hexyl, heptyl, and octyl groups.

The hydrocarbon ester is at least one selected from the group consisting of ethyl acetate, butyl acetate, and ethyl propionate, preferably ethyl acetate.

By using at least one selected from the group consisting of ethyl acetate, butyl acetate, and ethyl propionate as the hydrocarbon ester, the penetration between cellulose molecules is encouraged. As a result, the fluffy feel of the cellulosic particles further improves.

Ketone

A ketone is a compound having at least one carbonyl group and in which only a hydrocarbon group binds to the carbonyl group.

The number of carbonyl groups that the ketone contains may be one or two, preferably one.

The number of carbon atoms that the ketone contains may be one or more and eight or fewer, preferably one or more and five or fewer, more preferably one or more and three or fewer.

The hydrocarbon group that the ketone contains may be a saturated hydrocarbon group, and examples include the methyl, ethyl, n-propyl, isopropyl, n-butyl, isobutyl, pentyl, hexyl, heptyl, and octyl groups.

The ketone is at least one selected from the group consisting of methyl ethyl ketone, methyl isobutyl ketone, and acetone, preferably methyl ethyl ketone.

By using at least one selected from the group consisting of methyl ethyl ketone, methyl isobutyl ketone, and acetone as the ketone, the penetration between cellulose molecules is encouraged. As a result, the fluffy feel of the cellulosic particles further improves.

At Least One of the Amount of the Hydrocarbon Ester and the Amount of the Ketone

At least one of the amount of the hydrocarbon ester and the amount of the ketone is 3 ppm or more and 1000 ppm or less and may be 10 ppm or more and 500 ppm or less, preferably 15 ppm or more and 200 ppm or less, more preferably 20 ppm or more and 100 ppm or less. It should be noted that at least one of the amount of the hydrocarbon ester and the amount of the ketone is an amount relative to the cellulosic particles. The "ppm" is by mass.

The amount of hydrocarbon ester and that of ketone are measured using a gas chromatograph. An example of a gas chromatograph that can be used is Shimadzu Corporation's Nexis GC-2030. The measurement procedure is as follows.

Sets of cellulosic particles in which the amount of hydrocarbon ester is 3 ppm, 10 ppm, 100 ppm, 500 ppm, 1000 ppm, and 2000 ppm are each prepared. Then these sets of cellulosic particles are subjected to measurement with a gas chromatograph, and a calibration curve for calculating the amount of hydrocarbon ester is constructed.

Sets of cellulosic particles in which the amount of ketone is 3 ppm, 10 ppm, 100 ppm, 500 ppm, 1000 ppm, and 2000 ppm are each prepared. Then these sets of cellulosic particles are subjected to measurement with a gas chromatograph, and a calibration curve for calculating the amount of ketone is constructed.

Then the cellulosic particles of interest are subjected to measurement with the gas chromatograph, and the amounts of hydrocarbon ester and ketone are calculated based on the calibration curves.

Extra Constituents

The cellulosic particles according to this exemplary embodiment may contain extra constituents. If the cellulosic particles have the coating layer described later herein, the extra constituents are contained in the core particle, which is covered with the coating layer.

Examples of extra constituents include plasticizers, flame retardants, compatibilizers, release agents, light stabilizers, weathering agents, coloring agents, pigments, modifiers, anti-dripping agents, antistatic agents, anti-hydrolysis agents, fillers, reinforcing agents (glass fiber, carbon fiber, talc, clay, mica, glass flakes, milled glass, glass beads, crystalline silica, alumina, silicon nitride, aluminum nitride, boron nitride, etc.), acid acceptors for preventing acetic acid release (oxides, such as magnesium oxide and aluminum oxide; metal hydroxides, such as magnesium hydroxide, calcium hydroxide, aluminum hydroxide, and hydrotalcite; calcium carbonate; talc; etc.), and reactive trapping agents (e.g., epoxy compounds, acid anhydride compounds, carbodiimides, etc.).

The amounts of the extra constituents may be 0% by mass or more and 5% by mass or less for each constituent in relation to the total amount of the cellulosic particles (or core particles). In this context, "0% by mass" means that the extra constituent is not contained.

Cellulosic Particles Having a Coating Layer

The cellulosic particles according to this exemplary embodiment may be cellulosic particles having a core particle that contains cellulose as its base constituent (hereinafter also referred to as a cellulosic core particle) and a coating layer that covers the core particle and contains at least one selected from the group consisting of a fatty acid, a fatty acid metallic salt, and an amino acid compound (hereinafter also referred to as "cellulosic particles having a coating layer").

In this configuration, the cellulosic particles according to this exemplary embodiment assume a sponge-like structure, or a structure having spaces throughout, as a result of the carboxylic acid moiety of the fatty acid or fatty acid metallic salt being anchored by interacting with hydroxyl groups on the surface of the cellulose and the aliphatic groups, facing outward, repelling one another. The amino acid enhances flexibility as a result of its hydroxyl group becoming anchored by interacting with hydroxyl groups on the surface of the cellulose and its amino acid portion having a flexible structure. The fluffy feel of the cellulosic particles, therefore, further improves.

Core Particle

The core particle contains cellulose as its base constituent.

The cellulose contained in the core particle is synonymous with the cellulose described above, and possible and preferred ranges are also the same as described above.

Coating Layer

The coating layer contains at least one selected from the group consisting of a fatty acid, a fatty acid metallic salt, and an amino acid compound.

- Fatty Acid

A fatty acid is a linear-chain or branched saturated or unsaturated fatty acid. The fatty acid may be a mixture of a saturated fatty acid and an unsaturated fatty acid.

The fatty acid may be a fatty acid having 16 or more and 22 or fewer carbon atoms (C16 to C22; preferably a C18 to C20 fatty acid). Specific examples of C16 to C22 linear-chain fatty acids include behenic acid, arachidic acid, and palmitic acid.

The amount of the fatty acid may be 2% by mass or more and 15% by mass or less, preferably 5% by mass or more and 10% by mass or less, of the cellulosic particles as a whole.

By using a C16 to C22 fatty acid as the fatty acid, it is easier to cover the surface of the core particle. As a result, it is more likely that the number of hydroxyl groups exposed on the surface of the cellulosic particles is small.

- Fatty Acid Metallic Salt

A fatty acid metallic salt is a linear-chain or branched saturated or unsaturated fatty acid metallic salt. The fatty acid metallic salt may be a mixture of a saturated fatty acid metallic salt and an unsaturated fatty acid metallic salt.

Examples of fatty acid metallic salts include metallic salts of C16 to C22 (preferably C18 to C20) fatty acids. Examples of metallic salts of C16 to C22 fatty acids include metallic salts of stearic acid, metallic salts of behenic acid, and metallic salts of palmitic acid.

An example of a metal in a fatty acid metallic salt is a divalent metal.

Examples of metals in linear-chain fatty acid metallic salts include magnesium, calcium, aluminum, barium, and zinc.

The amount of the fatty acid metallic salt may be 2% by mass or more and 15% by mass or less, preferably 5% by mass or more and 10% by mass or less, of the cellulosic particles as a whole.

The number of carbon atoms in the fatty acid may be 16 or more and 22 or fewer, and the number of carbon atoms in the fatty acid metallic salt may be 16 or more and 22 or fewer.

By using C16 to C22 compounds as the fatty acid and the fatty acid metallic salt, the length is long enough that the aliphatic group produces its full effect and short enough that the affinity for the surface of the cellulosic particles is not impaired due to too strong hydrophobicity. As a result, the fluffy feel of the cellulosic particles further improves.

The fatty acid may be a saturated fatty acid, and the fatty acid metallic salt may be a saturated fatty acid metallic salt.

When the fatty acid and fatty acid metallic salt are a saturated fatty acid and a saturated fatty acid metallic salt, binding between aliphatic groups caused by the opening of an unsaturated portion is prevented. As a result, the fluffy feel of the cellulosic particles further improves.

- Amino Acid Compound

"Amino acid compounds" refers to amino acids and amino acid derivatives.

Examples of amino acid compounds include lauryl leucine, lauryl arginine, and myristyl leucine.

The amount of the amino acid compound may be 2% by mass or more and 10% by mass or less of the cellulosic particles as a whole.

Cellulosic particles having a coating layer may have an intermediate layer between the core particle and the coating layer. The intermediate layer, furthermore, may contain at least one selected from the group consisting of a polyamine compound, a polyquaternium, a polysaccharide compound, and a polyacrylic acid.

When the cellulosic particles have an intermediate layer, the affinity between the coating layer and the cellulosic particles is stronger. As a result, the fluffy feel of the cellulosic particles further improves.

"Polyamine compound" is a generic term for aliphatic hydrocarbons having two or more primary amino groups.

Examples of polyamine compounds include a polyalkyleneimine, polyallylamine, polyvinylamine, and polylysine.

The polyalkyleneimine may be a polyalkyleneimine possessing a constituent unit having a C1 to C6 (preferably C1 to C4, more preferably C1 or C2), preferably polyethyleneimine, for improved biodegradability.

Examples of polyallylamines include homopolymers or copolymers of allylamine, allylamine amidosulfate, diallylamine, dimethylallylamine, etc.

Examples of polyvinylamines include polyvinylamines manufactured by hydrolyzing poly(N-vinylformamide) with an alkali, and a specific example is Mitsubishi Chemical's "PVAM-0595B."

The polylysine may be polylysine extracted from a natural substance, may be polylysine produced by a transformed microorganism, or may be chemically synthesized polylysine.

The amount of the polyamine compound may be 0.2% by mass or more and 2% by mass or less of the cellulosic particles as a whole.

Examples of polyquaterniums include polyquaternium-6, polyquaternium-7, polyquaternium-10, polyquaternium-11, polyquaternium-51, polyquaternium-61, and polyquaternium-64.

The amount of the polyquaternium may be 0.2% by mass or more and 2% by mass or less of the cellulosic particles as a whole.

The amount of the polyacrylic acid may be 0.2% by mass or more and 2% by mass or less of the cellulosic particles as a whole.

Amounts of Constituents in the Coating and Intermediate Layers

The total amount of the fatty acid, fatty acid metallic salt, and amino acid compound relative to the entire coating layer may be 90% by mass or more and 100% by mass or less, preferably 95% by mass or more and 100% by mass or less.

The total amount of the polyamine compound, polyquaternium, polysaccharide compound, and polyacrylic acid relative to the entire intermediate layer may be 90% by mass or more and 100% by mass or less, preferably 95% by mass or more and 100% by mass or less.

External Additive

The cellulosic particles according to this exemplary embodiment may have inorganic particles as an external additive.

When the cellulosic particles have inorganic particles as an external additive, the inorganic particles are able to freely move up and down, following the flexibility of the surface of the cellulosic particles. As a result, the fluffy feel of the cellulosic particles further improves.

An example of an external additive is at least one selected from the group consisting of silicon-containing compound particles and metal oxide particles.

"Silicon-containing compound particles" indicates particles containing silicon.

The silicon-containing compound particles may be particles of silicon or may be particles containing silicon and one or more other elements.

The silicon-containing compound particles may be silica particles. The silica particles can be any particles containing silica, or $SiO_2$, as their base constituent and may be crystalline or amorphous. The silica particles, furthermore, may be particles manufactured from a silicon compound, such as waterglass or an alkoxysilane, as a raw material or may be particles obtained by crushing quartz.

As for metal oxides, oxides of metals other than silicon can be applied.

Examples of metal oxides include zinc oxide, magnesium oxide, iron oxide, and aluminum oxide.

The volume-average particle diameter of the external additive may be 1 nm or more and 100 nm or less, preferably 5 nm or more and 30 nm or less, for texture (specifically, feel when touched) reasons.

The volume-average particle diameter of the external additive is measured in the same manner as the volume-average particle diameter of the cellulose.

The amount of the external additive may be 0.1% by mass or more and 2% by mass or less of the mass of the cellulosic particles (the cellulosic particles without the external additive on them) as a whole.

Volume-Average Particle Diameter and Upper Geometric Standard Deviation by Number GSDv

The volume-average particle diameter of the cellulosic particles according to this exemplary embodiment may be 3 $\mu$m or more and less than 10 $\mu$m, preferably 4 $\mu$m or more and 9 $\mu$m or less, more preferably 5 $\mu$m or more and 8 $\mu$m or less.

When the volume-average particle diameter is 3 $\mu$m or more, the fluffy feel tends to be better because in that case the amount of deformation is large enough that the cellulosic particles can exhibit their flexibility.

When the volume-average particle diameter is less than 10 $\mu$m, a deterioration in feel and rugged feel caused by too large particle diameters are not felt, and the fluffy feel tends to be better. By virtue of a moderately large surface area, furthermore, biodegradation, which starts at the surface, is likely to progress uniformly, and biodegradability tends to be excellent.

The upper geometric standard deviation by number GSDv of the cellulosic particles according to this exemplary embodiment may be 1.0 or greater and 1.7 or less, preferably 1.0 or greater and 1.5 or less, more preferably 1.0 or greater and 1.3 or less.

When the GSDv is 1.0 or greater and 1.7 or less, hardness caused by fine particles and rugged feel caused by coarse particles are reduced, and the fluffy feel tends to improve.

The inhibition of biodegradation (which starts at the surface) by coarse particles (large particles greater than 10 $\mu$m) is also unlikely to occur.

The volume-average particle diameter and the upper geometric standard deviation GSDv of the cellulosic particles are measured as follows.

Particle diameters are measured using the LS particle size distribution analyzer "Beckman Coulter LS13 320 (Beckman Coulter)," and the cumulative distribution of particle diameters is plotted as a function of volume starting from the smallest diameter; then the particle diameter at which the cumulative percentage is 50% is determined as the volume-average particle diameter.

Separately, the cumulative distribution of particle diameters is plotted as a function of volume starting from the smallest diameter; the particle diameter at which the cumulative percentage is 50% is defined as the number-average particle diameter, D50v, and the particle diameter at which the cumulative percentage is 84% is defined as particle diameter D84v by number. The upper geometric standard deviation by number GSDv is calculated according to the equation GSDv = $(D84v/D50v)^{1/2}$.

The sphericity of the cellulosic particles according to this exemplary embodiment may be 0.7 or greater, preferably 0.8 or greater, more preferably 0.9 or greater.

When the sphericity is 0.7 or greater, the particles are likely to uniformly deform in all directions, and the fluffy feel tends to improve. Decomposition by microorganisms, furthermore, proceeds along the most efficient path, from the surface toward the inner core, and biodegradability tends to be excellent.

Sphericity is given by (circumference of the equivalent circle)/(circumference) [(circumference of a circle having the same projected area as the particle's image)/(circumference of the particle's projected image)]. Specifically, sphericity is a value measured by the following method.

First, the cellulosic particles of interest are sampled by aspiration in such a manner that the sample will form a flat stream, and this flat stream is photographed with a flash to capture the figures of the particles in a still image; then the sphericity is determined by analyzing the particle images using a flow particle-image analyzer (Sysmex Corp. FPIA-3000). The number of particles sampled in determining the sphericity is 3500.

If the cellulosic particles have an external additive, the cellulosic particles of interest are dispersed in water containing a surfactant, then the dispersion is sonicated to give cellulosic particles from which the external additive has been removed, and the resulting cellulosic particles are used as the subject of measurement.

Surface Smoothness

The surface smoothness of the cellulosic particles according to this exemplary embodiment may be 50% or more, preferably 60% or more and 99% or less, more preferably 70% or more and 98% or less.

When the smoothness is 50% or more, the expression of local hardness following surface irregularities is reduced, and the fluffy feel tends to improve. Biodegradability, furthermore, tends to be excellent; biodegrading microorganisms include species relatively large in size, and the particle surface in that case is accessible even to such large microorganisms.

The surface smoothness is measured through a procedure as described below.

An SEM image (magnification, 5,000 times) of the cellulosic particles, taken with a scanning electron microscope (SEM), is observed, and the smoothness M of the individual cellulosic particles is calculated according to the equation below. The arithmetic mean of the smoothness values M of ten or more randomly chosen cellulosic particles is reported as the surface smoothness. The closer the value of the smoothness M is to 1, the closer the surface of the cellulosic particles is to smoothness.

$$M = (1-(S3)/(S2)) \times 100$$

In this equation, S2 indicates the area that the cellulosic particle occupies in the image (projected area), and S3 indicates, when the cellulosic particle in the image and a circle having a projected area equal to S2 are superimposed, the sum of "the area outside the outline of the circle having a projected area equal to S2 and inside the outline of the cellulosic particle in the image" and "the area inside the outline of the circle having a projected area equal to S2 and outside the outline of the cellulosic particle in the image."

The method for superimposing the cellulosic particle in the image and a circle having a projected area equal to S2 is as follows.

The superposition is carried out in such a manner that when the cellulosic particle in the image and the circle having a projected area equal to S2 are superimposed, the area of the region shared by the two images (the area inside the outline of the circle having a projected area equal to S2 and inside the outline of the cellulosic particle in the image) is maximized.

Method for Manufacturing the Cellulosic Particles

An example of a method for manufacturing the cellulosic particles according to this exemplary embodiment is as follows.

Cellulosic Particle (core particle) Production Step

(1) First, cellulose acylate solution A is prepared by dissolving a cellulose acylate in organic solvent A.

(2) Then cellulose acylate solution B is prepared by adding cellulose acylate solution A into a calcium carbonate dispersion, in which calcium carbonate has been dispersed in water, and stirring the resulting mixture.

(3) Then cellulose acylate solution C is prepared by adding cellulose acylate solution B to a mixed solution of carboxymethylcellulose, organic solvent B, and water and rapidly stirring the resulting mixture.

(4) Then sodium hydroxide is added to cellulose acylate solution C. Subsequently, cellulose acylate dispersion C is heated to remove water-dissolving organic solvents A and B, and hydrochloric acid is added to cause the formation of cellulose acylate particles. Then the cellulose acylate particles are isolated by filtration, and the isolated cellulose acylate particles are dispersed in water; in this manner, a cellulose acylate particle dispersion is prepared.

(5) Then a cellulosic particle suspension is prepared by adding sodium hydroxide to the cellulose acylate particle dispersion and subsequently saponifying the cellulose acylate particles by warming the cellulose acylate particle dispersion in a weakly alkaline environment with stirring.

(6) Then the pH of the cellulosic particle suspension is adjusted to near neutral (e.g., 6.5 or higher and 7 or lower) by adding hydrochloric acid to the suspension, and subsequently isolation by filtration and washing in purified water of the cellulosic particles are repeated. After the electrical conductivity of the filtrate reaches 10 $\mu$s/cm or less, the isolated cellulosic particles are dried.

Organic solvent A may be a hydrocarbon ester, and organic solvent B may be a ketone.

The amounts of hydrocarbon ester and ketone contained can be adjusted by changing the amount of the hydrocarbon ester used as organic solvent A and that of the ketone used as organic solvent B added.

In the foregoing, a cellulose acylate is a cellulose derivative in which at least one of the hydroxy groups of cellulose has been replaced with an aliphatic acyl group (acylated). Specifically, a cellulose acylate is a cellulose derivative in which at least one of the hydroxy groups of cellulose has been replaced with -CO-$R^{AC}$ ($R^{AC}$ represents an aliphatic hydrocarbon group.).

Intermediate Layer and Coating Layer Formation Step

If cellulosic particles having a coating layer are manufactured, a step of forming the coating layer (coating layer formation step) may be included after the above cellulosic particle (core particle) production step.

If the coating layer formation step is carried out, the coating layer is formed using the particles obtained through the above cellulosic particle (core particle) production step as the core particles.

First, an aqueous dispersion in which the core particles have been dispersed is prepared. Before preparing the aqueous dispersion, the core particles may be cleaned with an acid.

Then the aqueous dispersion in which the core particles have been dispersed and an aqueous solution containing the compound that will form the intermediate layer are mixed together. Through that, the intermediate layer is formed, for example as a result of reaction between hydroxyl groups in the resin contained in the core particles and amine sites, carboxyl groups, amino groups, or any other moiety of the compound that will form the intermediate layer or as a result of hydrogen bonding between hydroxyl groups. Then the aqueous dispersion in which the core particles with the intermediate layer formed thereon are dispersed and an emulsion containing the compound that will form the coating layer are mixed together. Through that, the coating layer is formed.

If no intermediate layer is formed, the coating layer is formed by mixing together an aqueous dispersion in which the core particles obtained through the above cellulosic particle (core particle) production step have been dispersed and an emulsion containing the compound that will form the coating layer.

Then, from the mixture, the cellulosic particles having a coating layer are removed. The removal of the cellulosic particles having a coating layer is performed by, for example, filtering the mixture. The removed cellulosic particles having a coating layer may be washed with water. Through that, unreacted surface-treating polymers can be eliminated. Then, by drying the cellulosic particles having a coating layer, cellulosic particles according to this exemplary embodiment are obtained.

Addition Step

To the resulting cellulosic particles, an external additive may be added.

An example of an addition step is a treatment in which the external additive is added to the cellulosic particles using equipment like a mixing mill, V-blender, Henschel mixer, or Lödige mixer.

Applications

Applications of the cellulosic particles according to this exemplary embodiment include granular materials for use as cosmetics, a rolling agent, an abrasive, a scrubbing agent, display spacers, a material for bead molding, light-diffusing particles, a resin-strengthening agent, a refractive index control agent, a biodegradation accelerator, a fertilizer, water-absorbent particles, toner particles, and anti-blocking particles.

An application of the cellulosic particles according to this exemplary embodiment may be cosmetics.

In particular, an application of the cellulosic particles according to this exemplary embodiment may be a cosmetic additive.

The cellulosic particles according to this exemplary embodiment are superior in flexibility; when they are used as a cosmetic additive, therefore, it is likely that the spreading and hardness of the cosmetic on the skin to which the cosmetic is applied are good.

The cellulosic particles according to this exemplary embodiment can be applied as cosmetic additives, for example to base makeup cosmetics (e.g., foundation primer, concealer, foundation, and face powder); makeup cosmetics (e.g., lipstick, lip gloss, lip liner, blusher, eyeshadow, eyeliner, mascara, eyebrow powder, nail products, and nail care cosmetics); and skincare cosmetics (e.g., face wash, facial cleanser, toner, milky lotion, serum, face packs, face masks, and cosmetics for the care of the eye and mouth areas).

In particular, the resin particles according to this exemplary embodiment may be used as a cosmetic additive to makeup cosmetics because flexibility and biodegradability are required in such an application.

Examples

Examples will now be described, but no aspect of the present disclosure is limited to these examples. In the following description, "parts" and "%" are all by mass unless stated otherwise.

Preparation of Materials

The following materials are prepared.

Cellulose Acylates

- CA-1: Daicel Corporation's "L50," diacetyl cellulose, weight-average molecular weight = 8,000
- CA-2: Daicel Corporation's "L20," diacetyl cellulose, weight-average molecular weight = 47,000
- CA-3: Eastman Chemical "CAP482-20," cellulose acetate propionate, weight-average molecular weight = 75,000
- CA-4: Eastman Chemical "CAB381-20," cellulose acetate butyrate, weight-average molecular weight = 70,000
- CA-5: Eastman Chemical "CA398-6," diacetyl cellulose, weight-average molecular weight = 35,000

Coating Layer Formation Materials

Fatty Acids

- ST-1: NOF Corporation's "NAA-222S," behenic acid (saturated fatty acid), the number of carbon atoms = 22
- ST-2: NOF Corporation's "NAA-180," stearic acid (saturated fatty acid), the number of carbon atoms = 18
- ST-3: Miyoshi Oil & Fat Co., Ltd.'s "Palmitic Acid 98," palmitic acid (saturated fatty acid), the number of carbon atoms = 16
- ST-4: NOF Corporation's "NAA-142," myristic acid (saturated fatty acid), the number of carbon atoms = 14
- ST-5: Tokyo Chemical Industry Co., Ltd.'s "Lignoceric Acid," lignoceric acid (saturated fatty acid), the number of carbon atoms = 24
- ST-6: NOF Corporation's "EXTRA OS-85," oleic acid (unsaturated fatty acid with a degree of unsaturation of 1), the number of carbon atoms = 18
- ST-7: NOF Corporation's "Linoleic Acid 90," linoleic acid (unsaturated fatty acid with a degree of unsaturation of 2), the number of carbon atoms = 18

Fatty Acid Metallic Salts

- ST-8: NOF Corporation's "CALCIUM STEARATE VEGETABLE," calcium stearate (saturated fatty acid metallic salt), the number of carbon atoms = 18
- ST-9: NOF Corporation's "MAGNESIUM STEARATE S," magnesium stearate (saturated fatty acid metallic salt), the number of carbon atoms = 18
- ST-10: Nitto Chemical Industry Co., Ltd.'s "CS-7," calcium behenate (saturated fatty acid metallic salt), the number of carbon atoms = 22
- ST-11: NOF Corporation's "NONSOUL PK-1," potassium palmitate (saturated fatty acid metallic salt), the number of carbon atoms = 16
- ST-12: NOF Corporation's "POWDER BASE M," zinc myristate (saturated fatty acid metallic salt), the number of carbon atoms = 14
- ST-13: Tokyo Chemical Industry Co., Ltd.'s "Calcium Lignocerate," calcium lignocerate (saturated fatty acid metallic salt), the number of carbon atoms = 24
- ST-14: NOF Corporation's "NONSOUL ON-1N," sodium oleate (unsaturated fatty acid metallic salt with a degree of unsaturation of 1), the number of carbon atoms = 18
- ST-15: Nitto Chemical Industry Co., Ltd.'s "BS-5," barium linoleate (unsaturated fatty acid metallic salt with a degree of unsaturation of 2), the number of carbon atoms = 18 Amino Acid Compounds
- ST-16: Ajinomoto Co., Inc.'s "AMIHOPE LL," lauroyl lysine

- ST-17: Yoneyama Yakuhin Kogyo Co., Ltd.'s "Glycylglycine," glycylglycine
- ST-18: New Japan Chemical Co., Ltd.'s "DL-ALANINE," alanine

Silane Compound

- ST-19: Shin-Etsu Chemical Co., Ltd.'s "KBE-3083," octyltriethoxysilane

Intermediate Layer Formation Materials

Polyamine Compounds

- AA-1: Nippon Shokubai Co., Ltd.'s "PEI-1500," polyethyleneimine
- AA-2: BASF Japan Ltd.'s "Dehyquart H81," PEG-15 cocopolyamine
- AA-3: Ichimaru Pharcos Co., Ltd.'s "Polylysine 10," poly-ε-lysine Polyquaterniums
- AA-4: Nouryon Japan K.K.'s "CELQUAT SC230M," polyquaternium 10
- AA-5: BASF Japan Ltd.'s "Luviquat PQ11AT1," polyquaternium 11 Polysaccharide Compounds
- AA-6: Sumitomo Pharma Food & Chemical Co., Ltd.'s "GLYLOID 6C," *Tamarindus Indica* seed gum
- AA-7: Sumitomo Pharma Food & Chemical Co., Ltd.'s "RHABALL GUM CG-M," cationized guar gum

Arginine

- AA-8: Ajinomoto Co., Inc.'s "L-Arginine, C grade," arginine Polysaccharide Compound
- AA-9: Koyo Chemical Co., Ltd.'s "KOYO Chitosan FLA-40," chitosan Dextrin
- AA-10: San-ei Sucrochemical Co., Ltd.'s "NSD300A," dextrin Polysaccharide Compound
- AA-11: Hayashibara Co., Ltd.'s "PULLULAN (cosmetic grade)," pullulan Polyacrylic Acids
- AA-12: Toagosei Co., Ltd.'s "JURYMER AC-10H," a polyacrylic acid, weight-average molecular weight = 150,000
- AA-13: Toagosei Co., Ltd.'s "JURYMER AC-10SH," a polyacrylic acid, weight-average molecular weight = 1,000,000

External Additives

- EA-1: Wacker Asahikasei Silicone Co., Ltd.'s "HDK N20," silica particles, volume-average particle diameter = 200 nm
- EA-2: Wacker Asahikasei Silicone Co., Ltd.'s "HDK T30," silica particles, volume-average particle diameter = 300 nm

The volume-average particle diameters of the external additives are measured through the same procedure as the volume-average particle diameters of the cellulosic particles.

Examples 1 to 64 and Comparative Examples 1 to 8 (Example 19 is not included)

Cellulose Acylate Formation

The cellulose acylate of the species and amount indicated in Tables 1-1 and 1-3 is dissolved in ethyl acetate, which is a hydrocarbon ester, in the amount (parts) indicated in Tables 1-1 and 1-3. The resulting solution, solution A, is added to a dispersion in which calcium carbonate in the amount (parts) indicated in Tables 1-1 and 1-3 has been dispersed in 500 parts by mass of purified water, and the resulting mixture is stirred for 5 hours.

Then the resulting solution, solution B, is added to a dispersion in which carboxymethylcellulose in the amount (parts) indicated in Tables 1-1 and 1-3 and methyl ethyl ketone, which is a ketone, in the amount (parts) indicated in Tables 1-1 and 1-3 have been dispersed in 800 parts by mass of purified water, and the resulting mixture is stirred for 10 minutes using a high-speed emulsifier.

Then sodium hydroxide in the amount (parts) indicated in Tables 1-1 and 1-3 is added to the resulting solution, solution C, the resulting mixture is stirred for the time indicated in Tables 1-1 and 1-3 at 80°C to remove the ethyl acetate and methyl ethyl ketone, and subsequently diluted hydrochloric acid in the amount (parts) indicated in Tables 1-1 and 1-3 is added to dissolve the calcium carbonate and form cellulose acylate particles. Then the particles are isolated by filtration, and the isolated particles are dispersed again in purified water to give a slurry of cellulose acylate particles.

Saponification of Cellulose Acylate Particles

A 20% aqueous solution of sodium hydroxide in the amount (parts) indicated in Tables 1-1 and 1-3 is added to 500 parts by mass of the slurry of cellulose acylate particles (solids content, 50 parts by mass), and saponification is performed by stirring the resulting mixture at the reaction temperature and for the time indicated in Tables 1-1 and 1-3 to form cellulosic particles.

Then hydrochloric acid is added dropwise to the resulting slurry of cellulosic particles until the pH of the slurry reaches the pH in Tables 1-1 and 1-3. Subsequently, the slurry is filtered, the residue is washed with an excess of purified water, and the filtration and washing are repeated until the electrical conductivity of the filtrate reaches 10 $\mu$s/cm or less. The final cake of residue is filtered, and the resulting residue is lyophilized to give cellulosic particles (core particles).

Surface Treatment

In certain Examples and Comparative Examples, the cellulosic particles obtained through the saponification of cellulose acylate particles are used as core particles, and the core particles are subjected to the surface treatment described below.

The cake of residue after the repeated filtration and washing until the electrical conductivity of the filtrate reaches 10 $\mu$s/cm or less is reslurried with purified water to give a slurry of the core particles.

Then the intermediate layer formation material of the type and amount indicated in Tables 1-2-1, 1-2-2, 1-4-1, and 1-4-2 is added to 500 parts by mass of the slurry of the core particles (solids content, 50 parts by mass), and the resulting mixture is stirred for 3 hours at 30°C. Through that, an intermediate layer is formed on the surface of the core particles.

Then an emulsified form of the coating layer formation material of the type and amount indicated in Tables 1-2-1, 1-2-2, 1-4-1, and 1-4-2 is added to the slurry of core particles having an intermediate layer, and the resulting mixture is stirred for 24 hours. Through that, a coating layer is formed on the surface of the core particles having an intermediate layer.

Then the slurry of core particles having intermediate and coating layers is filtered, the residue is washed with purified water, and the resulting slurry is filtered again. This operation is repeated, and when the electrical conductivity of the filtrate reaches 10 $\mu$s/cm or less, the residue is lyophilized to give cellulosic particles having intermediate and coating layers. In addition, the treated surface of the cellulosic particles having intermediate and coating layers is smoothened by stirring the particles using FM Mixer (FM40, Nippon Coke & Engineering) for 3 hours at a frequency of 2000 min$^{-1}$ while the mixer temperature is maintained at 25°C.

Then the external additive of the type and amount (parts) indicated in Tables 1-2-1, 1-2-2, 1-4-1, and 1-4-2 is added to 100 parts of the cellulosic particles having intermediate and coating layers, and the materials are mixed together using a mixing mill (WONDER CRUSHER, Osaka Chemical) to give cellulosic particles having an external additive.

In certain Examples and Comparative Examples, cellulosic particles composed of a core particle and a coating layer formed thereon without an intermediate layer or cellulosic particles composed of a core particle, a coating layer formed thereon without an intermediate layer, and a subsequently added external additive are produced.

Table 1-1

| | Particle number | Cellulosic core particles | | | | | | | | | | | | |
| | | Cellulose acylate particles | | | | | | | | | Saponification | | | |
| | | Cellulose acylate | | Calcium carbonate | Ethyl acetate | Carboxymethylcellulose | Methyl ethyl ketone | Sodium hydroxide | Stirring time | Diluted hydrochloric acid | 20% aqueous solution of sodium hydroxide | Reaction temperature | Stirring time | pH |
| | | Compound | Parts by mass | Parts by mass | Parts by mass | Parts by mass | Parts mass | Parts by mass | hr | Parts by mass | Parts by mass | °C | hours | - |
| Example 1 | PTC-1 | CA1 | 200 | 60 | 1250 | 5 | 400 | 10 | 10 | 20 | 20 | 40 | 12 | 7 |
| Example 2 | PTC-2 | CA2 | 200 | 60 | 1250 | 5 | 400 | 10 | 10 | 20 | 20 | 40 | 12 | 7 |
| Example 3 | PTC-3 | CA3 | 200 | 60 | 1250 | 5 | 400 | 10 | 10 | 20 | 20 | 40 | 12 | 7 |
| Example 4 | PTC-4 | CA4 | 200 | 60 | 1250 | 5 | 400 | 10 | 10 | 20 | 20 | 40 | 12 | 7 |
| Example 5 | PTC-5 | CA5 | 200 | 60 | 1250 | 5 | 400 | 10 | 10 | 20 | 20 | 40 | 12 | 7 |
| Example 6 | PTC-6 | CA1 | 200 | 60 | 1050 | 5 | 320 | 10 | 10 | 20 | 20 | 40 | 12 | 7 |
| Example 7 | PTC-7 | CA1 | 200 | 60 | 1500 | 5 | 500 | 10 | 10 | 20 | 20 | 40 | 12 | 7 |
| Example 8 | PTC-8 | CA1 | 200 | 60 | 1250 | 7 | 400 | 10 | 10 | 20 | 20 | 40 | 12 | 7 |
| Example 9 | PTC-9 | CA1 | 200 | 60 | 1250 | 9 | 400 | 10 | 10 | 20 | 20 | 40 | 12 | 7 |
| Example 10 | PTC-10 | CA1 | 200 | 60 | 1250 | 4 | 400 | 10 | 10 | 20 | 20 | 40 | 12 | 7 |
| Example 11 | PTC-11 | CA1 | 200 | 60 | 1250 | 2 | 400 | 10 | 10 | 20 | 20 | 40 | 12 | 7 |
| Example 12 | PTC-12 | CA1 | 200 | 50 | 1250 | 5 | 400 | 10 | 10 | 20 | 20 | 40 | 12 | 7 |

(continued)

| | Particle number | Cellulosic core particles | | | | | | | | | | | | |
| | | Cellulose acylate particles | | | | | | | | | Saponification | | | |
| | | Cellulose acylate | | Calcium carbonate | Ethyl acetate | Carboxymethylcellulose | Methyl ethyl ketone | Sodium hydroxide | Stirring time | Diluted hydrochloric acid | 20% aqueous solution of sodium hydroxide | Reaction temperature | Stirring time | pH |
| | | Compound | Parts by mass | Parts by mass | Parts by mass | Parts by mass | Parts mass | Parts by mass | hr | Parts by mass | Parts by mass | °C | hours | - |
| Example 13 | PTC-13 | CA1 | 200 | 40 | 1250 | 5 | 400 | 10 | 7 | 20 | 20 | 40 | 12 | 7 |
| Example 14 | PTC-14 | CA1 | 200 | 60 | 1250 | 5 | 400 | 10 | 10 | 20 | 25 | 40 | 12 | 7 |
| Example 15 | PTC-15 | CA1 | 200 | 60 | 1250 | 5 | 400 | 10 | 10 | 20 | 15 | 40 | 12 | 7 |
| Example 16 | PTC-16 | CA1 | 200 | 60 | 1250 | 5 | 400 | 10 | 10 | 20 | 15 | 40 | 12 | 7 |
| Example 17 | PTC-17 | CA1 | 200 | 60 | 1250 | 5 | 400 | 7 | 10 | 20 | 20 | 40 | 12 | 7 |
| Example 18 | PTC-18 | CA1 | 200 | 60 | 1250 | 5 | 400 | 4 | 10 | 20 | 20 | 40 | 12 | 7 |
| Example 20 | PTC-20 | CA1 | 200 | 60 | 1250 | 5 | 400 | 10 | | 20 | 20 | 40 | 12 | 7 |
| Example 21 | PTC-21 | CA1 | 200 | 60 | 1250 | 5 | 400 | 10 | | 20 | 20 | 40 | 12 | 7 |
| Example 22 | PTC-22 | CA1 | 200 | 60 | 1250 | 5 | 400 | 10 | | 20 | 20 | 40 | 12 | 7 |
| Example 23 | PTC-23 | CA1 | 200 | 60 | 1250 | 5 | 400 | 10 | | 20 | 20 | 40 | 12 | 7 |
| Example 24 | PTC-24 | CA1 | 200 | 60 | 1250 | 5 | 400 | 10 | | 20 | 20 | 40 | 12 | 7 |
| Example 25 | PTC-25 | CA1 | 200 | 60 | 1250 | 5 | 400 | 10 | | 20 | 20 | 40 | 12 | 7 |

(continued)

| | Particle number | Cellulosic core particles | | | | | | | | | | | | |
| | | Cellulose acylate particles | | | | | | | | | Saponification | | | |
| | | Cellulose acylate | | Calcium carbonate | Ethyl acetate | Carboxymethylcellulose | Methyl ethyl ketone | Sodium hydroxide | Stirring time | Diluted hydrochloric acid | 20% aqueous solution of sodium hydroxide | Reaction temperature | Stirring time | pH |
| | | Compound | Parts by mass | Parts by mass | Parts by mass | Parts by mass | Parts mass | Parts by mass | hr | Parts by mass | Parts by mass | °C | hours | - |
| Example 26 | PTC-26 | CA1 | 200 | 60 | 1250 | 5 | 400 | 10 | | 20 | 20 | 40 | 12 | 7 |
| Example 27 | PTC-27 | CA1 | 200 | 60 | 1250 | 5 | 400 | 10 | | 20 | 20 | 40 | 12 | 7 |
| Example 28 | PTC-28 | CA1 | 200 | 60 | 1250 | 5 | 400 | 10 | | 20 | 20 | 40 | 12 | 7 |
| Example 29 | PTC-29 | CA1 | 200 | 60 | 1250 | 5 | 400 | 10 | | 20 | 20 | 40 | 12 | 7 |
| Example 30 | PTC-30 | CA1 | 200 | 60 | 1250 | 5 | 400 | 10 | | 20 | 20 | 40 | 12 | 7 |
| Example 31 | PTC-31 | CA1 | 200 | 60 | 1250 | 5 | 400 | 10 | | 20 | 20 | 40 | 12 | 7 |
| Example 32 | PTC-32 | CA1 | 200 | 60 | 1250 | 5 | 400 | 10 | | 20 | 20 | 40 | 12 | 7 |
| Example 33 | PTC-33 | CA1 | 200 | 60 | 1250 | 5 | 400 | 10 | | 20 | 20 | 40 | 12 | 7 |
| Example 34 | PTC-34 | CA1 | 200 | 60 | 1250 | 5 | 400 | 10 | | 20 | 20 | 40 | 12 | 7 |
| Example 35 | PTC-35 | CA1 | 200 | 60 | 1250 | 5 | 400 | 10 | | 20 | 20 | 40 | 12 | 7 |
| Example 36 | PTC-36 | CA1 | 200 | 60 | 1250 | 5 | 400 | 10 | | 20 | 20 | 40 | 12 | 7 |
| Example 37 | PTC-37 | CA1 | 200 | 60 | 1250 | 5 | 400 | 10 | | 20 | 20 | 40 | 12 | 7 |

| | | Cellulosic core particles | | | | | | | | | | | | |
|---|---|---|---|---|---|---|---|---|---|---|---|---|---|---|
| | | Cellulose acylate particles | | | | | | | | | Saponification | | | |
| | Particle number | Cellulose acylate | | Calcium carbonate | Ethyl acetate | Carboxymethylcellulose | Methyl ethyl ketone | Sodium hydroxide | Stirring time | Diluted hydrochloric acid | 20% aqueous solution of sodium hydroxide | Reaction temperature | Stirring time | pH |
| | | Compound | Parts by mass | Parts by mass | Parts by mass | Parts by mass | Parts mass | Parts by mass | hr | Parts by mass | Parts by mass | °C | hours | - |
| Example 38 | PTC-38 | CA1 | 200 | 60 | 1250 | 5 | 400 | 10 | | 20 | 20 | 40 | 12 | 7 |
| Example 39 | PTC-39 | CA1 | 200 | 60 | 1250 | 5 | 400 | 10 | | 20 | 20 | 40 | 12 | 7 |

Table 1-2-1

| | Surface treatment | | | | | | |
|---|---|---|---|---|---|---|---|
| | Cellulosic core particles | Intermediate layer formation material | | Coating layer formation material | | External additive | |
| | Parts by mass | Compound | Parts by mass | Compound | Parts by mass | Compound | Parts by mass |
| Example 1 | 50 | | | | | | |
| Example 2 | 50 | | | | | | |
| Example 3 | 50 | | | | | | |
| Example 4 | 50 | | | | | | |
| Example 5 | 50 | | | | | | |
| Example 6 | 50 | | | | | | |
| Example 7 | 50 | | | | | | |
| Example 8 | 50 | | | | | | |
| Example 9 | 50 | | | | | | |
| Example 10 | 50 | | | | | | |
| Example 11 | 50 | | | | | | |
| Example 12 | 50 | | | | | | |
| Example 13 | 50 | | | | | | |
| Example 14 | 50 | | | | | | |
| Example 15 | 50 | | | | | | |
| Example 16 | 50 | | | | | | |
| Example 17 | 50 | | | | | | |
| Example 18 | 50 | | | | | | |
| Example 20 | 50 | | | ST-2 | 5 | | |
| Example 21 | 50 | | | ST-3 | 5 | | |
| Example 22 | 50 | | | ST-4 | 5 | | |
| Example 23 | 50 | | | ST-5 | 5 | | |
| Example 24 | 50 | | | ST-6 | 5 | | |
| Example 25 | 50 | | | ST-7 | 5 | | |
| Example 26 | 50 | | | ST-8 | 5 | | |
| Example 27 | 50 | | | ST-9 | 5 | | |
| Example 28 | 50 | | | ST-10 | 5 | | |
| Example 29 | 50 | | | ST-11 | 5 | | |
| Example 30 | 50 | | | ST-12 | 5 | | |
| Example 31 | 50 | | | ST-13 | 5 | | |
| Example 32 | 50 | | | ST-14 | 5 | | |
| Example 33 | 50 | | | ST-15 | 5 | | |
| Example 34 | 50 | | | ST-16 | 5 | | |
| Example 35 | 50 | | | ST-17 | 5 | | |
| Example 36 | 50 | | | ST-18 | 5 | | |
| Example 37 | 50 | | | ST-19 | 5 | | |

(continued)

| | Surface treatment | | | | | | |
|---|---|---|---|---|---|---|---|
| | Cellulosic core particles | Intermediate layer formation material | | Coating layer formation material | | External additive | |
| | Parts by mass | Compound | Parts by mass | Compound | Parts by mass | Compound | Parts by mass |
| Example 38 | 50 | | | ST-8 | 3 | | |
| Example 39 | 50 | | | ST-8 | 7 | | |

Table 1-2-2

| | Particle characteristics | | | | | | | |
|---|---|---|---|---|---|---|---|---|
| | Particle diameter | GSDv | Sphericity | Surface smoothness | Mn | Percentage biodegradability | Hydrocarbon ester content | Ketone content |
| | μm | - | - | % | - | % | ppm | ppm |
| Example 1 | 7.5 | 1.4 | 0.92 | 90 | 58000 | 97 | 25 | 20 |
| Example 2 | 7.8 | 1.38 | 0.92 | 90 | 47000 | 98 | 20 | 18 |
| Example 3 | 7.4 | 1.42 | 0.94 | 92 | 52000 | 96 | 22 | 22 |
| Example 4 | 7.8 | 1.35 | 0.94 | 91 | 78000 | 98 | 25 | 18 |
| Example 5 | 6.5 | 1.34 | 0.88 | 88 | 31000 | 97 | 27 | 22 |
| Example 6 | 7.4 | 1.44 | 0.91 | 91 | 58000 | 96 | 4 | 4 |
| Example 7 | 7.6 | 1.38 | 0.93 | 92 | 58000 | 96 | 995 | 993 |
| Example 8 | 3.3 | 1.55 | 0.88 | 85 | 56000 | 95 | 27 | 29 |
| Example 9 | 2.8 | 1.61 | 0.89 | 81 | 57000 | 96 | 28 | 27 |
| Example 10 | 9.5 | 1.21 | 0.93 | 92 | 58000 | 97 | 29 | 26 |
| Example 11 | 10.2 | 1.22 | 0.92 | 88 | 58000 | 97 | 30 | 24 |
| Example 12 | 7.2 | 1.68 | 0.96 | 90 | 57000 | 97 | 31 | 29 |
| Example 13 | 7.8 | 1.73 | 0.87 | 87 | 56000 | 95 | 33 | 19 |
| Example 14 | 7.3 | 1.31 | 0.99 | 93 | 57000 | 94 | 30 | 23 |
| Example 15 | 7.2 | 1.29 | 0.75 | 92 | 58000 | 96 | 28 | 27 |
| Example 16 | 7.3 | 1.35 | 0.68 | 88 | 58000 | 95 | 27 | 25 |

(continued)

| | Particle characteristics | | | | | | | |
|---|---|---|---|---|---|---|---|---|
| | Particle diameter | GSDv | Sphericity | Surface smoothness | Mn | Percentage biodegradability | Hydrocarbon ester content | Ketone content |
| | μm | - | - | % | - | % | ppm | ppm |
| Example 17 | 7.2 | 1.56 | 0.81 | 54 | 59000 | 96 | 28 | 28 |
| Example 18 | 7 | 1.66 | 0.8 | 48 | 58000 | 96 | 29 | 25 |
| Example 20 | 7.7 | 1.55 | 0.91 | 91 | 57000 | 92 | 26 | 22 |
| Example 21 | 8 | 1.44 | 0.92 | 90 | 56000 | 91 | 25 | 26 |
| Example 22 | 7.8 | 1.43 | 0.91 | 86 | 58000 | 92 | 28 | 29 |
| Example 23 | 7.7 | 1.45 | 0.89 | 85 | 57000 | 91 | 27 | 28 |
| Example 24 | 7.8 | 1.55 | 0.88 | 87 | 58000 | 92 | 28 | 26 |
| Example 25 | 7.7 | 1.43 | 0.88 | 85 | 56000 | 93 | 26 | 29 |
| Example 26 | 7.9 | 1.34 | 0.93 | 90 | 58000 | 92 | 25 | 27 |
| Example 27 | 7.7 | 1.38 | 0.93 | 91 | 57000 | 91 | 24 | 25 |
| Example 28 | 7.8 | 1.35 | 0.92 | 91 | 58000 | 93 | 29 | 26 |
| Example 29 | 7.7 | 1.33 | 0.93 | 92 | 56000 | 92 | 28 | 28 |
| Example 30 | 7.6 | 1.42 | 0.88 | 87 | 55000 | 92 | 27 | 22 |
| Example 31 | 7.8 | 1.38 | 0.87 | 88 | 56000 | 91 | 30 | 29 |
| Example 32 | 7.8 | 1.44 | 0.87 | 86 | 58000 | 90 | 27 | 28 |
| Example 33 | 7.7 | 1.41 | 0.88 | 87 | 57000 | 91 | 29 | 25 |
| Example 34 | 7.6 | 1.35 | 0.91 | 90 | 58000 | 92 | 25 | 28 |
| Example 35 | 7.7 | 1.39 | 0.92 | 91 | 57000 | 92 | 28 | 26 |
| Example 36 | 7.8 | 1.38 | 0.92 | 91 | 58000 | 91 | 29 | 26 |
| Example 37 | 7.7 | 1.35 | 0.87 | 87 | 58000 | 92 | 38 | 23 |
| Example 38 | 7.6 | 1.35 | 0.93 | 92 | 59000 | 91 | 36 | 25 |

(continued)

| | Particle characteristics | | | | | | | |
|---|---|---|---|---|---|---|---|---|
| | Particle diameter | GSDv | Sphericity | Surface smoothness | Mn | Percentage biodegradability | Hydrocarbon ester content | Ketone content |
| | μm | - | - | % | - | % | ppm | ppm |
| Example 39 | 7.7 | 1.38 | 0.93 | 92 | 56000 | 93 | 35 | 27 |

Table 1-3

| | Particle number | Cellulosic core particles | | | | | | | | | | | | |
| | | Cellulose acylate particles | | | | | | | | | Saponification | | | |
| | | Cellulose acylate | | Calcium carbonate | Ethyl acetate | Carboxymethylcellulose | Methyl ethyl ketone | Sodium hydroxide | Stirring time | Diluted hydrochloric acid | 20% aqueous solution of sodium hydroxide | Reaction temperature | Stirring time | pH |
| | | Compound | Parts by mass | Parts by mass | Parts by mass | Parts by mass | Parts by mass | Parts by mass | hr | Parts by mass | Parts by mass | °C | hours | - |
| Example 40 | PTC-40 | CA1 | 200 | 60 | 1250 | 5 | 400 | 10 | | 20 | 20 | 40 | 12 | 7 |
| Example 41 | PTC-41 | CA1 | 200 | 60 | 1250 | 5 | 400 | 10 | | 20 | 20 | 40 | 12 | 7 |
| Example 42 | PTC-42 | CA1 | 200 | 60 | 1250 | 5 | 400 | 10 | | 20 | 20 | 40 | 12 | 7 |
| Example 43 | PTC-43 | CA1 | 200 | 60 | 1250 | 5 | 400 | 10 | | 20 | 20 | 40 | 12 | 7 |
| Example 44 | PTC-44 | CA1 | 200 | 60 | 1250 | 5 | 400 | 10 | | 20 | 20 | 40 | 12 | 7 |
| Example 45 | PTC-45 | CA1 | 200 | 60 | 1250 | 5 | 400 | 10 | | 20 | 20 | 40 | 12 | 7 |
| Example 46 | PTC-46 | CA1 | 200 | 60 | 1250 | 5 | 400 | 10 | | 20 | 20 | 40 | 12 | 7 |
| Example 47 | PTC-47 | CA1 | 200 | 60 | 1250 | 5 | 400 | 10 | | 20 | 20 | 40 | 12 | 7 |
| Example 48 | PTC-48 | CA1 | 200 | 60 | 1250 | 5 | 400 | 10 | | 20 | 20 | 40 | 12 | 7 |
| Example 49 | PTC-49 | CA1 | 200 | 60 | 1250 | 5 | 400 | 10 | | 20 | 20 | 40 | 12 | 7 |
| Example 50 | PTC-50 | CA1 | 200 | 60 | 1250 | 5 | 400 | 10 | | 20 | 20 | 40 | 12 | 7 |
| Example 51 | PTC-51 | CA1 | 200 | 60 | 1250 | 5 | 400 | 10 | | 20 | 20 | 40 | 12 | 7 |
| Example 52 | PTC-52 | CA1 | 200 | 60 | 1250 | 5 | 400 | 10 | | 20 | 20 | 40 | 12 | 7 |
| Example 53 | PTC-53 | CA1 | 200 | 60 | 1250 | 5 | 400 | 10 | | 20 | 20 | 40 | 12 | 7 |
| Example 54 | PTC-54 | CA1 | 200 | 60 | 1250 | 5 | 400 | 10 | | 20 | 20 | 40 | 12 | 7 |
| Example 55 | PTC-55 | CA1 | 200 | 60 | 1250 | 5 | 400 | 10 | | 20 | 20 | 40 | 12 | 7 |
| Example 56 | PTC-56 | CA1 | 200 | 60 | 1250 | 5 | 400 | 10 | | 20 | 20 | 40 | 12 | 7 |
| Example 57 | PTC-57 | CA1 | 200 | 60 | 1250 | 5 | 400 | 10 | | 20 | 20 | 40 | 12 | 7 |
| Example 58 | PTC-58 | CA1 | 200 | 60 | 1250 | 5 | 400 | 10 | | 20 | 20 | 40 | 12 | 7 |
| Example 59 | PTC-59 | CA1 | 200 | 60 | 1250 | 5 | 400 | 10 | | 20 | 20 | 40 | 12 | 7 |
| Example 60 | PTC-60 | CA1 | 200 | 60 | 1250 | 5 | 400 | 10 | | 20 | 20 | 40 | 12 | 7 |
| Example 61 | PTC-62 | CA1 | 200 | 60 | 900 | 5 | 400 | 10 | 10 | 20 | 20 | 40 | 12 | 7 |

| | Particle number | Cellulosic core particles | | | | | | | | | | | | |
|---|---|---|---|---|---|---|---|---|---|---|---|---|---|---|
| | | Cellulose acylate particles | | | | | | | | | Saponification | | | |
| | | Cellulose acylate | | Calcium carbonate | Ethyl acetate | Carboxymethylcellulose | Methyl ethyl ketone | Sodium hydroxide | Stirring time | Diluted hydrochloric acid | 20% aqueous solution of sodium hydroxide | Reaction temperature | Stirring time | pH |
| | | Compound | Parts by mass | Parts by mass | Parts by mass | Parts by mass | Parts by mass | Parts by mass | hr | Parts by mass | Parts by mass | °C | hours | - |
| Example 62 | PTC-63 | CA1 | 200 | 60 | 1250 | 5 | 270 | 10 | 10 | 20 | 20 | 40 | 12 | 7 |
| Example 63 | PTC-65 | CA1 | 200 | 60 | 1750 | 5 | 400 | 10 | 10 | 20 | 20 | 40 | 12 | 7 |
| Example 64 | PTC-66 | CA1 | 200 | 60 | 1250 | 5 | 600 | 10 | 10 | 20 | 20 | 40 | 12 | 7 |
| Comparative Example 1 | PTC-61 | CA1 | 200 | 60 | 900 | 5 | 270 | 10 | 10 | 20 | 20 | 40 | 12 | 7 |
| Comparative Example 2 | PTC-64 | CA1 | 200 | 60 | 1750 | 5 | 600 | 10 | 10 | 20 | 20 | 40 | 12 | 7 |
| Comparative Example 3 | PTC-57 | CA1 | 200 | 60 | 900 | 5 | 270 | 10 | 10 | 20 | 20 | 40 | 12 | 7 |
| Comparative Example 4 | PTC-68 | CA1 | 200 | 60 | 1750 | 5 | 600 | 10 | 10 | 20 | 20 | 40 | 12 | 7 |
| Comparative Example 5 | PTC-69 | CA1 | 200 | 60 | 900 | 5 | 270 | 10 | 10 | 20 | 20 | 40 | 12 | 7 |
| Comparative Example 6 | PTC-70 | CA1 | 200 | 60 | 1750 | 5 | 600 | 10 | 10 | 20 | 20 | 40 | 12 | 7 |
| Comparative Example 7 | PTC-71 | CA1 | 200 | 60 | 900 | 5 | 270 | 10 | 10 | 20 | 20 | 40 | 12 | 7 |
| Comparative Example 8 | PTC-72 | CA1 | 200 | 60 | 1750 | 5 | 600 | 10 | 10 | 20 | 20 | 40 | 12 | 7 |

EP 4 410 383 B1

Table 1-4-1

| | Surface treatment | | | | | | | |
| --- | --- | --- | --- | --- | --- | --- | --- | --- |
| | Cellulosic core particles | Intermediate layer formation material | | Coating layer formation material | | External additive | | |
| | Parts by mass | Compound | Parts by mass | Compound | Parts by mass | Compound | Parts by mass |
| Example 40 | 50 | AA-1 | 0.5 | ST-8 | 5 | | |
| Example 41 | 50 | AA-2 | 0.5 | ST-8 | 5 | | |
| Example 42 | 50 | AA-3 | 0.5 | ST-8 | 5 | | |
| Example 43 | 50 | AA-4 | 0.5 | ST-8 | 5 | | |
| Example 44 | 50 | AA-5 | 0.5 | ST-8 | 5 | | |
| Example 45 | 50 | AA-6 | 0.5 | ST-8 | 5 | | |
| Example 46 | 50 | AA-7 | 0.5 | ST-8 | 5 | | |
| Example 47 | 50 | AA-8 | 0.5 | ST-8 | 5 | | |
| Example 48 | 50 | AA-9 | 0.5 | ST-8 | 5 | | |
| Example 49 | 50 | AA-10 | 0.5 | ST-8 | 5 | | |
| Example 50 | 50 | AA-11 | 0.5 | ST-8 | 5 | | |
| Example 51 | 50 | AA-12 | 0.5 | ST-8 | 5 | | |
| Example 52 | 50 | AA-13 | 0.5 | ST-8 | 5 | | |
| Example 53 | 50 | AA-1 | 0.25 | ST-8 | 5 | | |
| Example 54 | 50 | AA-1 | 1.5 | ST-8 | 5 | | |
| Example 55 | 50 | AA-1 | 0.5 | ST-8 | 3 | | |
| Example 56 | 50 | AA-1 | 0.5 | ST-8 | 7 | | |
| Example 57 | 50 | AA-1 | 0.5 | ST-8 | 5 | EA-1 | 0.5 |
| Example 58 | 50 | AA-1 | 0.5 | ST-8 | 5 | EA-2 | 0.5 |
| Example 59 | 50 | AA-1 | 0.5 | ST-8 | 5 | EA-1 | 5 |
| Example 60 | 50 | | | ST-8 | 5 | EA-1 | 0.5 |
| Example 61 | 50 | | | | | | |
| Example 62 | 50 | | | | | | |
| Example 63 | 50 | | | | | | |
| Example 64 | 50 | | | | | | |
| Comparative Example 1 | 50 | | | | | | |
| Comparative Example 2 | 50 | | | | | | |
| Comparative Example 3 | 50 | | | ST-8 | 5 | | |
| Comparative Example 4 | 50 | | | ST-8 | 5 | | |
| Comparative Example 5 | 50 | AA-1 | 0.5 | ST-8 | 5 | | |
| Comparative Example 6 | 50 | AA-1 | 0.5 | ST-8 | 5 | | |
| Comparative Example 7 | 50 | AA-1 | 0.5 | ST-8 | 5 | EA-1 | 0.5 |
| Comparative Example 8 | 50 | AA-1 | 0.5 | ST-8 | 5 | EA-1 | 0.5 |

Table 1-4-2

| | Particle characteristics | | | | | | | |
| --- | --- | --- | --- | --- | --- | --- | --- | --- |
| | Particle diameter | GSDv | Sphericity | Surface smoothness | Mn | Percentage biodegradability | Hydrocarbon ester content | Ketone content |
| | μm | - | - | % | - | % | ppm | ppm |
| Example 40 | 7.8 | 1.41 | 0.94 | 93 | 58000 | 78 | 25 | 25 |
| Example 41 | 7.6 | 1.4 | 0.95 | 94 | 58000 | 77 | 27 | 29 |
| Example 42 | 7.5 | 1.38 | 0.94 | 93 | 58000 | 74 | 29 | 21 |
| Example 43 | 7.7 | 1.39 | 0.96 | 92 | 57000 | 78 | 25 | 28 |
| Example 44 | 7.8 | 1.42 | 0.95 | 91 | 58000 | 76 | 28 | 23 |
| Example 45 | 7.7 | 1.42 | 0.94 | 90 | 57000 | 72 | 23 | 27 |
| Example 46 | 7.8 | 1.38 | 0.95 | 90 | 58000 | 74 | 28 | 24 |
| Example 47 | 7.7 | 1.55 | 0.89 | 86 | 58000 | 78 | 25 | 26 |
| Example 48 | 7.6 | 1.58 | 0.88 | 87 | 59000 | 76 | 27 | 29 |
| Example 49 | 7.7 | 1.62 | 0.87 | 86 | 56000 | 75 | 28 | 33 |
| Example 50 | 7.8 | 1.58 | 0.9 | 90 | 58000 | 75 | 24 | 20 |
| Example 51 | 7.8 | 1.58 | 0.9 | 90 | 58000 | 74 | 29 | 39 |
| Example 52 | 7.8 | 1.58 | 0.9 | 90 | 59000 | 73 | 26 | 28 |
| Example 53 | 7.5 | 1.44 | 0.95 | 90 | 58000 | 73 | 27 | 27 |
| Example 54 | 7.4 | 1.38 | 0.94 | 91 | 56000 | 72 | 24 | 20 |
| Example 55 | 7.5 | 1.33 | 0.93 | 92 | 57000 | 71 | 25 | 26 |
| Example 56 | 7.7 | 1.32 | 0.94 | 93 | 58000 | 72 | 28 | 29 |
| Example 57 | 8 | 1.35 | 0.91 | 90 | 56000 | 68 | 26 | 33 |
| Example 58 | 8 | 1.37 | 0.91 | 90 | 55000 | 69 | 27 | 40 |
| Example 59 | 8.1 | 1.34 | 0.92 | 90 | 54000 | 68 | 28 | 35 |
| Example 60 | 8 | 1.35 | 0.91 | 90 | 55000 | 67 | 25 | 27 |
| Example 61 | 7.4 | 1.44 | 0.91 | 91 | 56000 | 68 | 2 | 30 |
| Example 62 | 7.4 | 1.44 | 0.91 | 91 | 58000 | 69 | 25 | 2 |
| Example 63 | 7.6 | 1.38 | 0.93 | 92 | 57000 | 68 | 1050 | 25 |

(continued)

| | Particle characteristics | | | | | | | |
|---|---|---|---|---|---|---|---|---|
| | Particle diameter | GSDv | Sphericity | Surface smoothness | Mn | Percentage biodegradability | Hydrocarbon ester content | Ketone content |
| | μm | - | - | % | - | % | ppm | ppm |
| Example 64 | 7.6 | 1.38 | 0.93 | 92 | 56000 | 69 | 30 | 1050 |
| Comparative Example 1 | 7.4 | 1.44 | 0.91 | 91 | 55000 | 69 | 2 | 2 |
| Comparative Example 2 | 7.6 | 1.38 | 0.93 | 92 | 56000 | 67 | 1050 | 1050 |
| Comparative Example 3 | 7.8 | 1.45 | 0.89 | 89 | 55000 | 67 | 2 | 2 |
| Comparative Example 4 | 7.6 | 1.38 | 0.89 | 87 | 56000 | 68 | 1050 | 1050 |
| Comparative Example 5 | 7.5 | 1.44 | 0.88 | 87 | 57000 | 66 | 2 | 2 |
| Comparative Example 6 | 7.3 | 1.42 | 0.87 | 88 | 56000 | 67 | 1050 | 1050 |
| Comparative Example 7 | 7.9 | 1.41 | 0.88 | 87 | 58000 | 68 | 2 | 2 |
| Comparative Example 8 | 8.1 | 1.43 | 0.89 | 88 | 57000 | 69 | 1050 | 1050 |

Comparative Examples 9 to 13

The following particles are used as cellulosic particles of these Comparative Examples.

Comparative Example 9: CELLULOBEADS D10 (Daito Kasei, cellulosic particles containing cellulose as their base constituent. No intermediate layer, no coating layer, and no external additive.)

Comparative Example 10: CELLUFLOW C25 (JNC, cellulosic particles containing cellulose as their base constituent. No intermediate layer, no coating layer, and no external additive.)

Comparative Example 11: CELLUFLOW T25 (JNC, cellulosic particles containing cellulose acetate as their base constituent. No intermediate layer, no coating layer, and no external additive.)

Comparative Example 12: OTS-0.5A CELLULOBEADS D10 (Daito Kasei, cellulosic particles having a core particle containing cellulose as its base constituent and a coating layer containing triethoxyoctylsilane. No external additive.)

Comparative Example 13: S-STM CELLULOBEADS D-5 (Daito Kasei, cellulosic particles having a core particle containing cellulose as its base constituent and a coating layer containing magnesium stearate. No external additive.)

Comparative Example 14

Cellulosic particles are obtained according to the procedure described in Example 1 in Japanese Patent No. 6921293. The specific production process is as follows.

An oil phase is prepared by dissolving 150 parts of cellulose acetate (trade name "CA-398-6," Eastman Chemical; the percentage of acetyl groups, 39.8%) in 1,350 parts of ethyl acetate (solubility in water, 8 g/100 g). A water phase is prepared by dissolving 100 parts of polyvinyl alcohol in 1,250 parts of deionized water. The oil phase is added to the prepared water phase, the two phases are mixed together, and the resulting mixture is stirred for 3 minutes at 1,000 rpm using a dissolver. The mixture is further stirred for 10 minutes at 2,000 rpm using a dissolver to give a suspension in which oil droplets are uniformly dispersed. The volume-average particle diameter of the oil droplets measured through observation under an optical microscope and image analysis is 18 $\mu$m.

While the resulting suspension is stirred at 500 rpm using a dissolver, 42,000 parts of deionized water is introduced over 90 minutes to give a resin particle dispersion. After filtration and washing, the resin particles are deflocculated in deionized water and stirred. The resin particles are collected by filtration and washed, and the washed resin particles are dispersed in 2,500 parts of deionized water. The pH is adjusted to 13.0 or lower by adding sodium hydroxide, and hydrolysis is performed through heating to 50°C. After the end of the hydrolysis, the dispersion is neutralized with hydrochloric acid. After filtration and washing, the product is deflocculated in deionized water. Then, after filtration and washing, drying and crushing are performed to give core beads having a median diameter (D50) of 9 $\mu$m.

Fifty grams of the resulting core beads and 1.5 g of zinc stearate (trade name "SPZ-100F," Sakai Chemical Industry; a powder of sheet-shaped particles; average particle diameter, 0.4 $\mu$m; thickness, 0.1 $\mu$m; aspect ratio, 3) are put into a small-sized mixer. The surface of the core beads is treated with the zinc stearate through dry mixing for 3 minutes to give resin beads.

The resulting resin beads are used as cellulosic particles of Comparative Example 14.

Comparative Example 15

Cellulosic particles are obtained according to the procedure described in Example 2 in Japanese Patent No. 6921293. The specific production process is as follows.

Resin beads are obtained in the same manner as in Example 1 in Japanese Patent No. 6921293, except that 2.5 g of magnesium stearate (trade name "SPX-100F," Sakai Chemical Industry; a powder of sheet-shaped particles; average particle diameter, 0.7 $\mu$m; thickness, 0.1 $\mu$m; aspect ratio, 4) is used instead of the zinc stearate.

The resulting resin beads are used as cellulosic particles of Comparative Example 15.

Table 2

| | | Product name | Manufacturer | Core particles | Anchoring agent | Surface treatment agent | External additive | Particle diameter | GSDv | Sphericity | Surface smoothness | Mn | Percentage biodegradability | Hydrocarbon ester content | Ketone content |
|---|---|---|---|---|---|---|---|---|---|---|---|---|---|---|---|
| Comparative Example 9 | PT-C-10-1 | CELLULOBEADS D10 | Daito Kasei | Cellulose | None | None | None | 14 | 1.17 | 0.97 | 94 | 110000 | 79 | 0 | 1 |
| Comparative Example 10 | PT-C-10-2 | CELLUFLOW C25 | JNC | Cellulose | None | None | None | 10 | 1.86 | 0.97 | 88 | 45000 | 78 | 0 | 1250 |
| Comparative Example 11 | PT-C-10-3 | CELLUFLOW T25 | JNC | Cellulose acetate | None | None | None | 12 | 1.94 | 0.98 | 88 | 48000 | 17 | 0 | 1300 |
| Comparative Example 12 | PT-C-10-4 | OTS-0.5A CELLULOBEADS D-10 (Example 1 in Japanese Unexamined Patent Application Publication No. 2020-132616) | Daito Kasei | Cellulose | None | Triethoxyoctylsilane | None | 14 | 1.32 | 0.98 | 85 | 110000 | 25 | 0 | 1 |
| Comparative Example 13 | PT-C-10-5 | S-STM CELLULOBEADS D-5 (Example 2 in Japanese Unexamined Patent Application Publication No. 2020-132616) | Daito Kasei | Cellulose | None | Magnesium stearate | None | 10 | 1.86 | 0.97 | 56 | 110000 | 24 | 0 | 0 |
| | | Related art | | Core particles | Anchoring agent | Surface treatment agent | External additive | Particle diameter | GSDv | Sphericity | Surface smoothness | Mn | Percentage biodegradability | Hydrocarbon ester content | Ketone content |
| Comparative Example 14 | PT-C-11-1 | Example 1 in Japanese 6921293 | Patent No. | Cellulose | None | Zinc stearate | None | 9 | 1.45 | 0.96 | 92 | 33000 | 85 | 0 | 2 |

28

EP 4 410 383 B1

| | | Related art | | Core particles | Anchoring agent | Surface treatment agent | External additive | Particle diameter | GSDv | Sphericity | Surface smoothness | Mn | Percentage biodegradability | Hydrocarbon ester content | Ketone content |
|---|---|---|---|---|---|---|---|---|---|---|---|---|---|---|---|
| Comparative Example 15 | PT-C-11-2 | Example 2 in Japanese 6921293 | Patent No. | Cellulose | None | Magnesium stearate | None | 9 | 1.55 | 0.96 | 92 | 32000 | 88 | 0 | 2 |

Characteristics Evaluation

Particle Characteristics

For the cellulosic particles obtained in the Examples and Comparative Examples, the following particle characteristics are measured according to the methods described above.

- Volume-average particle diameter ("Particle diameter" in the tables)
- Upper geometric standard deviation by number ("GSDv" in the tables)
- Sphericity
- Surface smoothness
- Number-average molecular weight of cellulose ("Mn" in the tables)
- Amounts of hydrocarbon ester and ketone

Percentage Biodegradability

The 60-day percentage biodegradability in activated sludge of the cellulosic particles obtained in the Examples and Comparative Examples is measured by a method according to OECD 306F.

Cosmetics Evaluation

Production of Cosmetics

Of the cellulosic particles of the Examples and Comparative Examples, the cellulosic particles indicated in Tables 4-1 and 4-3 are used to produce a variety of cosmetics. Specifically, the following is performed.

Liquid Foundation

According to the formula presented in Table 3-1, liquid foundation is obtained by a known method.

Table 3-1 Liquid Foundation

| Formula | Compound | Product name (manufacturer) | Parts by mass |
|---|---|---|---|
| Particles | Particles | The cellulosic particles specified in Tables 4-1 and 4-3 | 10 |
| Ingredients other than the particles | Propylene glycol | Propylene Glycol JSQI (Dow Toray) | 5 |
| | Bentonite | OVWIL BR (Mizusawa Industrial Chemicals) | 1 |
| | Triethanolamine | Triethanolamine 99% (Dow Toray) | 1 |
| | Stearic acid | NAA172 (NOF) | 3 |
| | Stearyl alcohol | NAA45 (NOF) | 1 |
| | Liquid paraffin | MORESCO-VIOLESS (MORESCO) | 8 |
| | Isopropyl myristate | IPM-R (NOF) | 5 |
| | Petrolatum | NOMCORT W (Nisshin OilliO) | 2 |
| | Stearic acid monoglyceride | EXCEL 84 (Kao Chemicals) | 2 |
| | POE (20) stearyl ether | EMALEX 602 (Nihon Emulsion) | 1 |
| | Titanium oxide | NMR-1 (Sakai Chemical) | 8 |
| | Kaolin | BERACLAY 20061 AMAZONIAN WHITE CLAY (BERECA) | 5 |
| | Iron oxide | C33-128 Sun CROMA RED Iron Oxide (Sun Chemical) | 0.5 |
| | Preservative | OPTIPHEN HD (Ashland Japan) | 0.5 |
| | Fragrance | Bisabolol rac. (BASF Japan) | 0.3 |
| | Purified water | | 46.5 |

(continued)

| Formula | Compound | Product name (manufacturer) | Parts by mass |
|---|---|---|---|
| Total | | | 100 |

Milky Lotion

According to the formula presented in Table 3-2, a milky lotion is obtained by a known method.

Table 3-2 Milky Lotion

| Formula | Compound | Product name (manufacturer) | Parts by mass |
|---|---|---|---|
| Particles | Particles | As in the Example or Comparative Example | 2 |
| Ingredients other than the particles | Propylene glycol | Propylene Glycol JSQI (Dow Toray) | 5 |
| | Polyethylene glycol 1500 | PEG#1500 (NOF) | 3 |
| | Carboxy vinyl polymer | NTC-CARBOMER 380 (Nikko Chemicals) | 0.1 |
| | Triethanolamine | Triethanolamine 99% (Dow Toray) | 1 |
| | Stearic acid | NAA172 (NOF) | 2 |
| | Cetyl alcohol | NAA44 (NOF) | 1.5 |
| | Liquid paraffin | MORESCO-VIOLESS (MORESCO) | 10 |
| | Petrolatum | NOMCORT W (Nisshin OilliO) | 3 |
| | Glyceryl oleate | NIKKOL MGO (Nikko Chemicals) | 1 |
| | POE (20) sorbitan oleate | NIKKOL TO -0V (Nikko Chemicals) | 1 |
| | Preservative | OPTIPHEN HD (Ashland Japan) | 0.2 |
| | Fragrance | Bisabolol rac. (BASF Japan) | 0.1 |
| | Purified water | | 70.1 |
| Total | | | 100 |

Loose Powder

The ingredients listed in Table 3-3 are mixed together in a blender, the resulting mixture is milled in a mill, and then the resulting particles are sieved through a 250-$\mu$m mesh sieve to give a loose powder.

Table 3-3 Loose Powder

| Formula | Compound | Product name (manufacturer) | Parts by mass |
|---|---|---|---|
| Particles | Particles | The cellulosic particles specified in Tables 4-1 and 4-3 | 10 |
| Ingredients other than the particles | Talc | Talc CT-25 (Yamaguchi Mica) | 65 |
| | Kaolin | BERACLAY 20061 AMAZONIAN WHITE CLAY (BERECA) | 5 |
| | Titanium oxide | MKR-1 (Sakai Chemical) | 3 |
| | Zinc myristate | POWDER BASE M (NOF) | 5 |
| | Magnesium carbonate | Natrasorb HFB (Nouryon Japan) | 5 |
| | Sericite | Sericite FSE (Sanshin Mining Ind.) | 7 |
| Total | | | 100 |

Powder Foundation

According to the formula presented in Table 3-4, the particles and powders are mixed together, the binders are mixed together separately, the mixture of particles and powders is gradually added into the binders with stirring, and then the mixture is mixed to give powder foundation.

Table 3-4 Powder Foundation

| Formula | Compound | Product name (manufacturer) | Parts by mass |
|---|---|---|---|
| Particles | Particles | The cellulosic particles specified in Tables 4-1 and 4-3 | 8 |
| Powders other than the particles | Talc | Talc CT-25 (Yamaguchi Mica) | 52.5 |
| | Mica | Mica FA450 (Yamaguchi Mica) | 16 |
| | Titanium oxide | MKR-1 (Sakai Chemical) | 12 |
| | Black iron oxide | C33-134 Sun CROMA Black Iron Oxide (Sun Chemical) | 0.2 |
| | Red iron oxide | C33-128 Sun CROMA Red Iron Oxide (Sun Chemical) | 0.4 |
| | Yellow iron oxide | C33-210 Sun CROMA Yellow Iron Oxide (Sun Chemical) | 2.4 |
| Binders | Diisostearyl malate | Neosolue-DiSM (Nippon Fine Chemical) | 3 |
| | Caprylic/capric triglyceride | Caprylic/Capric Triglyceride (FUJIFILM Wako Pure Chemical) | 2 |
| | Neopentyl glycol dicaprate | NPDC (Kokyu Alcohol Kogyo) | 2 |
| | Pentylene glycol | DIOL PD (Kokyu Alcohol Kogyo) | 1.5 |
| Total | | | 100 |

Sunscreen Cream

According to the formula presented in Table 3-5, oil phase (1) is warmed to 50°C until dissolution, then oil phase (2) is added, and the two phases are mixed together. Water phase (2) is brought into dissolution and mixed with water phase (1). After the particles and the powders are added to the mixture of oil phases (1) and (2) and dispersed and mixed, emulsification is performed by gradually adding the mixture of water phases (1) and (2), giving a sunscreen cream.

Table 3-5 Sunscreen Cream

| Formula | Compound | Product name (manufacturer) | Parts by mass |
|---|---|---|---|
| Particles | Particles | The cellulosic particles specified in Tables 4-2 and 4-4 | 5 |
| Powders other than the particles | Quaternium-18 hectorite | SUMECTON-SAN (Kunimine Industries) | 1 |
| | Titanium oxide | MKR-1 (Sakai Chemical) | 8 |
| Oil phase (1) | Ethylhexyl methoxycinnamate | Uvinul MC80 (BASF Japan) | 4 |
| | t-Butyl methoxydibenzoylmethane | Eusolex 9030 (Merck KGaA) | 0.5 |
| | Bis-ethylhexyloxyphenol methoxyphenyl triazine | Tinosorb S (BASF Japan) | 2 |
| | Isopropyl sebacate | Isopropyl Sebacate (FUJIFILM Wako Pure Chemical) | 6 |
| | Caprylic/capric triglyceride | Caprylic/Capric Triglyceride (FUJIFILM Wako Pure Chemical) | 2 |

(continued)

| Formula | Compound | Product name (manufacturer) | Parts by mass |
|---|---|---|---|
| Oil phase (2) | Cetyl PEG/PPG-10/1 dimethicone | KF-6048 (Shin-Etsu Chemical) | 4 |
| | Sorbitan isostearate | EMALEX SPIS 100 (Nihon Emulsion) | 0.4 |
| | Cyclopentasiloxane | KF-995 (Shin-Etsu Chemical) | 16 |
| | Ethylhexylglycerin, glyceryl caprylate | NIKKOL NIKKOGUARD 88 (Nikko Chemicals) | 0.4 |
| Water phase (1) | PEG-240/HDI copolymer bis-decyltetradeceth-20 ether | ADEKA NOL GT 700 | 1 |
| | Glycerin | RG-CO-P (NOF) | 4 |
| | 1,3-Butylene glycol | HAISUGARCANE BG (Kokyu Alcohol Kogyo) | 4 |
| | Pentylene glycol | DIOL PD (Kokyu Alcohol Kogyo) | 1 |
| | Phenoxyethanol | Phenoxetol (Clariant Japan) | 0.3 |
| Water phase (2) | Magnesium sulfate | Magnesium Sulfate (FUJIFILM Wako Pure Chemical) | 0.3 |
| | Purified water | | 40.1 |
| Total | | | 100 |

All-in-One Gel

According to the formula presented in Table 3-6, water phases (1) and (2) are mixed together. Then oil phase (1) is mixed and added to the mixture of water phases (1) and (2). After oil phase (2) is warmed to 70°C, the particles are added to give a dispersion. The resulting dispersion is added to the mixture of water phases (1) and (2) and oil phase (1), and emulsification is performed by stirring and mixing the resulting mixture. After the neutralizing agent is added, the emulsion is stirred and cooled to give an all-in-one gel.

Table 3-6 All-in-One Gel

| Formula | Compound | Product name (manufacturer) | Parts by mass |
|---|---|---|---|
| Particles | Particles | The cellulosic particles specified in Tables 4-2 and 4-4 | 4 |
| Oil phase (1) | Xanthan gum | NOMCORT Z (The Nisshin OilliO Group) | 0.1 |
| | Hydrogenated lecithin | COATSOME NC-21 (NOF) | 0.1 |
| | Glycerin | RG-CO-P (NOF) | 5 |
| | Isopentyldiol | Isoprene Glycol (Kuraray) | 4 |
| Oil phase (2) | Polyglyceryl-10 isostearate | Sunsoft Q-18S-C (Taiyo Kagaku) | 1.2 |
| | Polyglyceryl-4 isostearate | NIKKOL Tetraglyn 1-SV (Nikko Chemicals) | 0.3 |
| | Behenyl alcohol | NAA-422 (NOF) | 1.8 |
| | Octyldodecanol | RISONOL 20SP (Kokyu Alcohol Kogyo) | 0.8 |
| | Cetyl ethylhexanoate | FineNeo-CIO (Nippon Fine Chemical) | 3.2 |
| | Squalane | NIKKOL Olive Squalane (Nikko Chemicals) | 0.6 |
| | Tocopherol | Tocopherol 100 (The Nisshin OilliO Group) | 0.6 |
| | Ethylhexylglycerin, glyceryl caprylate | NIKKOL NIKKOGUARD 88 (Nikko Chemicals) | 0.6 |

(continued)

| Formula | Compound | Product name (manufacturer) | Parts by mass |
|---|---|---|---|
| Water phase (1) | Carboxy vinyl polymer | NTC-CARBOMER 380 (Nikko Chemicals) | 0.4 |
| | Pentylene glycol | DIOL PD (Kokyu Alcohol Kogyo) | 1 |
| | Phenoxyethanol | Phenoxetol (Clariant Japan) | 0.3 |
| | Sodium dilauramidoglutamide lysine, water | Pellicer LB 100 (Asahi Kasei Finechem) | 0.1 |
| Water phase (2) | Citric acid | Citric Acid (FUJIFILM Wako Pure Chemical) | 0.1 |
| | Purified water | | 1.4 |
| Neutralizing agent A 10% aqueous solution of sodium hydroxide | | | |
| Total | | | 100 |

Foundation Primer

According to the formula presented in Table 3-7, the particles are dispersed in component A, and the resulting mixture is stirred. Component B is added, and the resulting mixture is stirred to give a foundation primer.

Table 3-7 Foundation Primer

| Formula | Compound | Product name (manufacturer) | Parts by mass |
|---|---|---|---|
| Particles | Particles | The cellulosic particles specified in Tables 4-2 and 4-4 | 10 |
| Component A | Dimethicone/PEG-10/15 crosspolymer, dimethicone | KSG-210 (Shin-Etsu Chemical) | 3.5 |
| | PEG-9 polydimethylsiloxyethyl dimethicone | KF-6028 (Shin-Etsu Chemical) | 2 |
| | Dimethicone | KF-7312K (Shin-Etsu Chemical) | 5 |
| | Isononyl isononanoate | KAK99 (Kokyu Alcohol Kogyo) | 4.5 |
| | Ethylhexyl methoxycinnamate | NOMCORT TAB (The Nisshin OilliO Group) | 10 |
| | Quaternium-18 hectorite | SUMECTON-SAN (Kunimine Industries) | 1.2 |
| | Dimethicone/vinyl dimethicone crosspolymer, dimethicone | KSG-16 (Shin-Etsu Chemical) | 5 |
| | Cyclomethicone | DOWSIL SH245 Fluid (Dow Toray) | 25 |
| Component B | 1,3-Butylene glycol | HAISUGARCANE BG (Kokyu Alcohol Kogyo) | 5 |
| | Sodium citrate | Trisodium Citrate (Jungbunzlauer International AG) | 2 |
| | Preservative | OPTIPHEN HD (Ashland Japan) | 0.3 |
| | Purified water | | 26.5 |
| Total | | | 100 |

Lip Primer

According to the formula presented in Table 3-8, component B is heated to 60°C and mixed. The particles are dispersed in the mixture, component A is added, heating in a microwave is performed until dissolution, the resulting solution is mixed, and then the resulting mixture is poured into a mold and cooled. The resulting solid is set into a lipstick case to give a lip primer.

Table 3-8 Lip Primer

| Formula | Compound | Product name (manufacturer) | Parts by mass |
|---|---|---|---|
| Particles | Particles | The cellulosic particles specified in Tables 4-2 and 4-4 | 10 |
| Component A | Ceresin | CERESIN #810 (Nikko Rika) | 4.27 |
| | Microcrystalline wax | Refined Microcrystalline Wax (Nikko Rika) | 1.55 |
| | Candelilla wax | Refined Candelilla Wax No. 1 (Nippon Wax) | 5.03 |
| | Paraffin | Refined Paraffin Wax (Nikko Rika) | 3.07 |
| Component B | Diisostearyl malate | Neosolue-DiSM (Nippon Fine Chemical) | 17.95 |
| | Dipentaerythrite fatty acid ester | COSMOL 168 EV (The Nisshin OilliO Group) | 6.22 |
| | Adsorption refined lanolin | SUPER STEROL LIQUID (Croda Japan) | 2.52 |
| | Liquid lanolin acetate | ACELAN SP (Croda Japan) | 13.34 |
| | Ethylhexylglyceryl | GLYMOIST (NOF) | 19.02 |
| | Liquid paraffin | HYDROBRITE 380 PO (Sonneborn) | 7.28 |
| | Isotridecyl isononanoate | KAK139 (Kokyu Alcohol Kogyo) | 3.21 |
| | Polyglyceryl-2 triisostearate | EMALEX TISG-2 (Nihon Emulsion) | 4.01 |
| | Methylphenyl polysiloxane | BELSIL PDM 20 (Wacker Asahikasei Silicone) | 2.41 |
| | Methylparaben | Nipagin M (Clariant Japan) | 0.07 |
| | Tocopherol | Tocopherol 100 (The Nisshin OilliO Group) | 0.05 |
| | Total | | 100 |

Body Powder

A body powder is obtained by mixing together the ingredients listed in Table 3-9 using a laboratory mixer.

Table 3-9 Body Powder

| Formula | Compound | Product name (manufacturer) | Parts by mass |
|---|---|---|---|
| Particles | Particles | The cellulosic particles specified in Tables 4-2 and 4-4 | 10 |
| Ingredients other than the particles | Talc | Talc CT-25 (Yamaguchi Mica) | 89.7 |
| | Fragrance | Bisabolol rac. (BASF Japan) | 0.3 |

Solid Powder Eyeshadow

According to the formula presented in Table 3-10, the particles and powders are mixed together, the binder is uniformly dissolved, the resulting solution is added to the powder mixture, the resulting mixture is further mixed, and then the resulting mixture is compression-molded to give a solid powder eyeshadow.

Table 3-10 Solid Powder Eyeshadow

| Formula | Compound | Product name (manufacturer) | Parts by mass |
|---|---|---|---|
| Particles | Particles | The cellulosic particles specified in Tables 4-2 and 4-4 | 51 |

(continued)

| Formula | Compound | Product name (manufacturer) | Parts by mass |
|---|---|---|---|
| Powders other than the particles | Mica | Talc CT-25 (Yamaguchi Mica) | 15 |
| | Sericite | Sericite FSE (Sanshin Mining Ind.) | 5 |
| | Pigment | Unipure Blue LC 621 (Sensient Technologies Japan) | 15 |
| | Pearl pigment | TWINCLEPEARL (Nihon Koken Kogyo | 10 |
| Binder | Methyl polysiloxane | BELSIL DM 10 (Wacker Asahikasei Silicone) | 2 |
| Others | Sorbitan sesquioleate | EMALEX SPO-150 (Nihon Emulsion) | 2 |
| Total | | | 100 |

Fluffy Feel Evaluations

The fluffy feel of the resulting cellulosic particles and cosmetics is evaluated as follows.

It should be noted that the greater the compression energy, described below, is, the greater the resilience after being pressed is, and thus the better the fluffy feel is; and the greater the work of recovery is, the better the recovery from compression is, and thus the better the fluffy feel is.

Evaluation of the Fluffy Feel of Cellulosic Particles

The cellulosic particles are packed into a 60-mm square and 20-mm deep SUS vat, and compression energy and the work of recovery are measured using a texture tester (KES-G5, Kato Tech).

Evaluation of the Fluffy Feel of Solid Cosmetics

For the loose powder, powder foundation, body powder, and solid powder eyeshadow, the fluffy feel is evaluated as follows.

Compression energy and the work of recovery are evaluated in the same manner as the cellulosic particles, except that the cosmetic is packed into the SUS vat.

Evaluation of the Fluffy Feel of Liquid or Cream Cosmetics

For the cosmetics other than the loose powder, powder foundation, body powder, and solid powder eyeshadow, the fluffy feel is evaluated as follows.

The cosmetic is applied to artificial skin (Exseal, As One), and compression energy and the work of recovery are evaluated using a texture tester (KES-G5, Kato Tech) on the coated surface.

Table 4-1

| | Particle number | Percentage biodegradability (%) | Fluffy feel | | | | | | | | | |
|---|---|---|---|---|---|---|---|---|---|---|---|---|
| | | | Particles | | Liquid foundation | | Milky lotion | | Loose powder | | Powder foundation | |
| | | | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) |
| Example 1 | PTC-1 | 97 | 2.25 | 0.77 | 0.22 | 0.07 | 0.21 | 0.07 | 2.13 | 0.72 | 2.02 | 0.69 |
| Example 2 | PTC-2 | 98 | 2.24 | 0.75 | 0.21 | 0.07 | 0.22 | 0.07 | 2.11 | 0.69 | 2.01 | 0.68 |
| Example 3 | PTC-3 | 96 | 2.21 | 0.74 | 0.22 | 0.07 | 0.22 | 0.08 | 2.16 | 0.71 | 1.99 | 0.67 |
| Example 4 | PTC-4 | 98 | 2.23 | 0.73 | 0.23 | 0.08 | 0.22 | 0.07 | 2.14 | 0.71 | 1.98 | 0.66 |
| Example 5 | PTC-5 | 97 | 1.88 | 0.59 | 0.15 | 0.04 | 0.17 | 0.04 | 1.87 | 0.55 | 1.81 | 0.55 |
| Example 6 | PTC-6 | 96 | 2.12 | 0.75 | 0.22 | 0.08 | 0.23 | 0.08 | 2.11 | 0.68 | 1.99 | 0.69 |
| Example 7 | PTC-7 | 96 | 2.21 | 0.71 | 0.23 | 0.07 | 0.21 | 0.07 | 2.08 | 0.67 | 1.97 | 0.69 |
| Example 8 | PTC-8 | 95 | 2.23 | 0.72 | 0.23 | 0.08 | 0.22 | 0.07 | 2.1 | 0.69 | 1.99 | 0.67 |
| Example 9 | PTC-9 | 96 | 1.89 | 0.59 | 0.17 | 0.04 | 0.16 | 0.04 | 1.87 | 0.56 | 1.79 | 0.54 |
| Example 10 | PTC-10 | 97 | 2.21 | 0.71 | 0.22 | 0.07 | 0.22 | 0.08 | 2.14 | 0.71 | 2.01 | 0.66 |
| Example 11 | PTC-11 | 97 | 1.92 | 0.58 | 0.17 | 0.04 | 0.17 | 0.05 | 1.88 | 0.57 | 1.77 | 0.52 |
| Example 12 | PTC-12 | 97 | 2.22 | 0.72 | 0.21 | 0.07 | 0.21 | 0.08 | 2.13 | 0.71 | 1.98 | 0.68 |
| Example 13 | PTC-13 | 95 | 1.87 | 0.57 | 0.18 | 0.04 | 0.17 | 0.04 | 1.85 | 0.56 | 1.78 | 0.53 |

| | Particle number | Percentage biodegradability (%) | Fluffy feel | | | | | | | | | |
|---|---|---|---|---|---|---|---|---|---|---|---|---|
| | | | Particles | | Liquid foundation | | Milky lotion | | Loose powder | | Powder foundation | |
| | | | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) |
| Example 14 | PTC-14 | 94 | 2.25 | 0.71 | 0.22 | 0.08 | 0.2 | 0.07 | 2.12 | 0.71 | 1.99 | 0.69 |
| Example 15 | PTC-15 | 96 | 2.23 | 0.71 | 0.23 | 0.08 | 0.21 | 0.07 | 2.11 | 0.68 | 1.98 | 0.67 |
| Example 16 | PTC-16 | 95 | 1.85 | 0.57 | 0.17 | 0.04 | 0.18 | 0.04 | 1.83 | 0.55 | 1.76 | 0.55 |
| Example 17 | PTC-17 | 96 | 2.22 | 0.71 | 0.22 | 0.07 | 0.2 | 0.08 | 2.25 | 0.69 | 2.01 | 0.69 |
| Example 18 | PTC-18 | 96 | 1.86 | 0.58 | 0.17 | 0.04 | 0.17 | 0.04 | 1.84 | 0.54 | 1.74 | 0.54 |
| Example 20 | PTC-20 | 92 | 2.66 | 0.89 | 0.36 | 0.1 | 0.28 | 0.15 | 2.58 | 1.11 | 2.33 | 0.97 |
| Example 21 | PTC-21 | 91 | 2.65 | 0.88 | 0.35 | 0.12 | 0.29 | 0.14 | 2.57 | 1.13 | 2.32 | 0.98 |
| Example 22 | PTC-22 | 92 | 2.35 | 0.65 | 0.27 | 0.08 | 0.22 | 0.1 | 2.11 | 0.99 | 2 | 0.77 |
| Example 23 | PTC-23 | 91 | 2.38 | 0.68 | 0.26 | 0.08 | 0.23 | 0.1 | 2.12 | 0.99 | 2.01 | 0.76 |
| Example 24 | PTC-24 | 92 | 2.36 | 0.67 | 0.27 | 0.07 | 0.22 | 0.09 | 2.11 | 0.98 | 2.02 | 0.74 |
| Example 25 | PTC-25 | 93 | 2.38 | 0.66 | 0.28 | 0.08 | 0.21 | 0.1 | 2.09 | 0.99 | 2.03 | 0.75 |
| Example 26 | PTC-26 | 92 | 2.68 | 0.89 | 0.35 | 0.12 | 0.28 | 0.13 | 2.58 | 1.12 | 2.34 | 0.97 |
| Example 27 | PTC-27 | 91 | 2.66 | 0.9 | 0.36 | 0.13 | 0.29 | 0.17 | 2.59 | 1.22 | 2.32 | 0.96 |

EP 4 410 383 B1

38

(continued)

| Particle number | Percentage biodegradability (%) | Fluffy feel | | | | | | | | | |
| | | Particles | | Liquid foundation | | Milky lotion | | Loose powder | | Powder foundation | |
| | | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) |
|---|---|---|---|---|---|---|---|---|---|---|---|
| Example 28 / PTC-28 | 93 | 2.67 | 0.88 | 0.36 | 0.15 | 0.27 | 0.15 | 2.59 | 1.12 | 2.34 | 0.96 |
| Example 29 / PTC-29 | 92 | 2.65 | 0.87 | 0.37 | 0.14 | 0.29 | 0.16 | 2.58 | 1.15 | 2.33 | 0.97 |
| Example 30 / PTC-30 | 92 | 2.36 | 0.65 | 0.25 | 0.08 | 0.21 | 0.09 | 2.12 | 0.99 | 2.01 | 0.73 |
| Example 31 / PTC-31 | 91 | 3.35 | 0.63 | 0.27 | 0.07 | 0.2 | 0.09 | 2.11 | 0.98 | 2.02 | 0.77 |
| Example 32 / PTC-32 | 90 | 2.39 | 0.64 | 0.28 | 0.08 | 0.22 | 0.1 | 2.12 | 0.99 | 2 | 0.75 |
| Example 33 / PTC-33 | 91 | 2.37 | 0.61 | 0.27 | 0.07 | 0.21 | 0.09 | 2.1 | 0.98 | 2.02 | 0.74 |
| Example 34 / PTC-34 | 92 | 2.68 | 0.89 | 0.36 | 0.13 | 0.28 | 0.17 | 2.58 | 1.12 | 2.32 | 0.97 |
| Example 35 / PTC-35 | 92 | 2.72 | 0.88 | 0.37 | 0.12 | 0.29 | 0.16 | 2.59 | 1.13 | 2.35 | 0.96 |
| Example 36 / PTC-36 | 91 | 2.65 | 0.87 | 0.35 | 0.13 | 0.28 | 0.16 | 2.59 | 1.12 | 2.36 | 0.96 |
| Example 37 / PTC-37 | 92 | 2.22 | 0.62 | 0.26 | 0.08 | 0.21 | 0.1 | 2.11 | 0.99 | 2.01 | 0.72 |
| Example 38 / PTC-38 | 91 | 2.67 | 0.87 | 0.34 | 0.12 | 0.28 | 0.15 | 2.59 | 1.13 | 2.34 | 0.97 |
| Example 39 / PTC-39 | 93 | 2.68 | 0.89 | 0.35 | 0.13 | 0.29 | 0.16 | 2.58 | 1.13 | 2.32 | 0.96 |

Table 4-2

| | Fluffy feel | | | | | | | | | | | |
|---|---|---|---|---|---|---|---|---|---|---|---|---|
| | Sunscreen cream | | All-in-one gel | | Foundation primer | | Lip primer | | Body powder | | Solid powder eyeshadow | |
| | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) |
| Example 1 | 0.35 | 0.09 | 0.21 | 0.07 | 2.13 | 0.72 | 2.02 | 0.69 | 2.25 | 0.71 | 2.45 | 0.81 |
| Example 2 | 0.33 | 0.09 | 0.22 | 0.07 | 2.11 | 0.69 | 2.01 | 0.68 | 2.22 | 0.69 | 2.38 | 0.78 |
| Example 3 | 0.34 | 0.09 | 0.22 | 0.08 | 2.16 | 0.71 | 1.99 | 0.67 | 2.23 | 0.67 | 2.41 | 0.77 |
| Example 4 | 0.33 | 0.08 | 0.22 | 0.07 | 2.14 | 0.71 | 1.98 | 0.66 | 2.23 | 0.67 | 2.38 | 0.76 |
| Example 5 | 0.28 | 0.06 | 0.17 | 0.04 | 1.87 | 0.55 | 1.81 | 0.55 | 1.89 | 0.59 | 2.11 | 0.54 |
| Example 6 | 0.34 | 0.09 | 0.23 | 0.08 | 2.11 | 0.68 | 1.99 | 0.69 | 2.24 | 0.69 | 2.38 | 0.77 |
| Example 7 | 0.33 | 0.08 | 0.21 | 0.07 | 2.08 | 0.67 | 1.97 | 0.69 | 2.35 | 0.67 | 2.41 | 0.73 |
| Example 8 | 0.34 | 0.09 | 0.22 | 0.07 | 2.1 | 0.69 | 1.99 | 0.67 | 2.21 | 0.67 | 2.39 | 0.72 |
| Example 9 | 0.29 | 0.06 | 0.16 | 0.04 | 1.87 | 0.56 | 1.79 | 0.54 | 1.88 | 0.57 | 2.09 | 0.57 |
| Example 10 | 0.33 | 0.08 | 0.22 | 0.08 | 2.14 | 0.71 | 2.01 | 0.66 | 2.22 | 0.68 | 2.38 | 0.76 |
| Example 11 | 0.28 | 0.06 | 0.17 | 0.05 | 1.88 | 0.57 | 1.77 | 0.52 | 1.87 | 0.55 | 2.09 | 0.56 |
| Example 12 | 0.34 | 0.09 | 0.21 | 0.08 | 2.13 | 0.71 | 1.98 | 0.68 | 2.21 | 0.69 | 2.38 | 0.77 |
| Example 13 | 0.28 | 0.06 | 0.17 | 0.04 | 1.85 | 0.56 | 1.78 | 0.53 | 1.87 | 0.59 | 2.08 | 0.55 |

(continued)

| Example | Sunscreen cream | | All-in-one gel | | Fluffy feel Foundation primer | | Lip primer | | Body powder | | Solid powder eyeshadow | |
|---|---|---|---|---|---|---|---|---|---|---|---|---|
| | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) |
| Example 14 | 0.35 | 0.09 | 0.2 | 0.07 | 2.12 | 0.71 | 1.99 | 0.69 | 2.21 | 0.66 | 2.37 | 0.76 |
| Example 15 | 0.33 | 0.08 | 0.21 | 0.07 | 2.11 | 0.68 | 1.98 | 0.67 | 2.19 | 0.68 | 2.36 | 0.75 |
| Example 16 | 0.27 | 0.06 | 0.18 | 0.04 | 1.83 | 0.55 | 1.76 | 0.55 | 1.87 | 0.57 | 2.08 | 0.55 |
| Example 17 | 0.34 | 0.09 | 0.2 | 0.08 | 2.25 | 0.69 | 2.01 | 0.69 | 2.15 | 0.67 | 2.33 | 0.76 |
| Example 18 | 0.28 | 0.06 | 0.17 | 0.04 | 1.84 | 0.54 | 1.74 | 0.54 | 1.86 | 0.54 | 2.07 | 0.55 |
| Example 20 | 0.56 | 0.14 | 0.28 | 0.14 | 2.65 | 0.9 | 2.22 | 0.99 | 2.53 | 1.03 | 2.69 | 1.02 |
| Example 21 | 0.55 | 0.15 | 0.29 | 0.13 | 2.65 | 0.89 | 2.23 | 0.98 | 2.52 | 1.01 | 2.7 | 1.01 |
| Example 22 | 0.39 | 0.09 | 0.24 | 0.1 | 2.23 | 0.67 | 2.01 | 0.75 | 2.33 | 0.87 | 2.43 | 0.88 |
| Example 23 | 0.37 | 0.08 | 0.23 | 0.11 | 2.35 | 0.69 | 1.99 | 0.76 | 2.34 | 0.88 | 2.41 | 0.89 |
| Example 24 | 0.38 | 0.09 | 0.24 | 0.1 | 2.33 | 0.68 | 1.99 | 0.77 | 2.35 | 0.86 | 2.41 | 0.88 |
| Example 25 | 0.37 | 0.08 | 0.23 | 0.1 | 2.34 | 0.69 | 2 | 0.76 | 2.34 | 0.87 | 2.42 | 0.87 |
| Example 26 | 0.57 | 0.14 | 0.29 | 0.14 | 2.64 | 0.9 | 2.21 | 0.97 | 2.51 | 1 | 2.71 | 1 |
| Example 27 | 0.55 | 0.16 | 0.29 | 0.15 | 2.63 | 0.91 | 2.23 | 0.98 | 2.52 | 1.01 | 2.69 | 1.01 |

| | Fluffy feel | | | | | | | | | | | |
|---|---|---|---|---|---|---|---|---|---|---|---|---|
| | Sunscreen cream | | All-in-one gel | | Foundation primer | | Lip primer | | Body powder | | Solid powder eyeshadow | |
| | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) |
| Example 28 | 0.54 | 0.13 | 0.29 | 0.15 | 2.65 | 0.88 | 2.22 | 0.99 | 2.51 | 1.03 | 2.7 | 1 |
| Example 29 | 0.58 | 0.15 | 0.28 | 0.14 | 2.64 | 0.88 | 2.21 | 0.98 | 2.52 | 1.04 | 2.68 | 1 |
| Example 30 | 0.36 | 0.08 | 0.24 | 0.11 | 2.32 | 0.69 | 1.98 | 0.78 | 2.35 | 0.88 | 2.43 | 0.88 |
| Example 31 | 0.38 | 0.09 | 0.23 | 0.09 | 2.32 | 0.69 | 2 | 0.75 | 2.36 | 0.87 | 2.42 | 0.89 |
| Example 32 | 0.39 | 0.08 | 0.23 | 0.1 | 2.31 | 0.69 | 2.01 | 0.76 | 2.35 | 0.87 | 2.41 | 0.87 |
| Example 33 | 0.38 | 0.09 | 0.24 | 0.1 | 2.33 | 0.69 | 2.01 | 0.77 | 2.35 | 0.88 | 2.42 | 0.88 |
| Example 34 | 0.55 | 0.13 | 0.29 | 0.14 | 2.64 | 0.89 | 2.21 | 0.98 | 2.55 | 1 | 2.68 | 1.02 |
| Example 35 | 0.57 | 0.14 | 0.29 | 0.14 | 2.63 | 0.88 | 2.21 | 0.97 | 2.52 | 1.01 | 2.67 | 1 |
| Example 36 | 0.58 | 0.15 | 0.28 | 0.14 | 2.63 | 0.87 | 2.19 | 0.98 | 2.51 | 1 | 2.66 | 1 |
| Example 37 | 0.39 | 0.08 | 0.23 | 0.11 | 2.33 | 0.68 | 1.99 | 0.77 | 2.36 | 0.87 | 2.41 | 0.89 |
| Example 38 | 0.56 | 0.15 | 0.28 | 0.14 | 2.65 | 0.87 | 2.23 | 0.99 | 2.51 | 1.02 | 2.71 | 1.01 |
| Example 39 | 0.57 | 0.14 | 0.29 | 0.13 | 2.64 | 0.88 | 2.21 | 0.98 | 2.51 | 1.01 | 2.7 | 1.01 |

Table 4-3

| | Particle number | Percentage biodegradability (%) | Fluffy feel | | | | | | | | | |
|---|---|---|---|---|---|---|---|---|---|---|---|---|
| | | | Particles | | Liquid foundation | | Milky lotion | | Loose powder | | Powder foundation | |
| | | | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) |
| Example 40 | PTC-40 | 78 | 3.15 | 1.01 | 0.44 | 0.19 | 0.38 | 0.24 | 2.88 | 1.45 | 2.88 | 1.55 |
| Example 41 | PTC-41 | 77 | 3.11 | 0.99 | 0.45 | 0.18 | 0.37 | 0.22 | 2.89 | 1.44 | 2.87 | 1.68 |
| Example 42 | PTC-42 | 74 | 3.12 | 1.01 | 0.41 | 0.18 | 0.37 | 0.24 | 2.89 | 1.45 | 2.86 | 1.58 |
| Example 43 | PTC-43 | 78 | 3.15 | 1 | 0.53 | 0.17 | 0.38 | 0.23 | 2.88 | 1.45 | 2.89 | 1.57 |
| Example 44 | PTC-44 | 76 | 3.03 | 0.99 | 0.43 | 0.18 | 0.37 | 0.22 | 2.89 | 1.47 | 2.87 | 1.55 |
| Example 45 | PTC-45 | 72 | 3.15 | 1.03 | 0.45 | 0.19 | 0.36 | 0.21 | 2.88 | 1.43 | 2.87 | 1.58 |
| Example 46 | PTC-46 | 74 | 3.11 | 1.03 | 0.42 | 0.18 | 0.37 | 0.22 | 2.89 | 1.45 | 2.88 | 1.57 |
| Example 47 | PTC-47 | 78 | 2.76 | 0.86 | 0.35 | 0.15 | 0.29 | 0.16 | 2.66 | 1.15 | 2.55 | 1.23 |
| Example 48 | PTC-48 | 76 | 3.09 | 1.01 | 0.43 | 0.17 | 0.37 | 0.23 | 2.9 | 1.44 | 2.89 | 1.56 |
| Example 49 | PTC-49 | 75 | 2.71 | 0.88 | 0.54 | 0.14 | 0.28 | 0.15 | 2.65 | 1.2 | 256 | 1.33 |
| Example 50 | PTC-50 | 75 | 3.08 | 1.01 | 0.43 | 0.18 | 0.36 | 0.23 | 2.88 | 1.48 | 2.88 | 1.57 |
| Example 51 | PTC-51 | 74 | 3.15 | 1.02 | 0.45 | 0.19 | 0.36 | 0.22 | 2.89 | 1.43 | 2.89 | 1.55 |
| Example 52 | PTC-52 | 73 | 3.13 | 0.99 | 0.42 | 0.17 | 0.37 | 0.23 | 2.9 | 1.43 | 2.87 | 1.56 |
| Example 53 | PTC-53 | 73 | 3.15 | 1.05 | 0.42 | 0.18 | 0.36 | 0.24 | 2.91 | 1.45 | 2.88 | 1.55 |
| Example 54 | PTC-54 | 72 | 3.15 | 1.03 | 0.43 | 0.19 | 0.37 | 0.23 | 2.88 | 1.43 | 2.89 | 1.54 |
| Example 55 | PTC-55 | 71 | 3.11 | 1.03 | 0.42 | 0.18 | 0.35 | 0.22 | 2.87 | 1.42 | 2.87 | 1.57 |
| Example 56 | PTC-56 | 72 | 3.12 | 1.02 | 0.42 | 0.19 | 0.36 | 0.23 | 2.85 | 1.42 | 2.86 | 1.56 |
| Example 57 | PTC-57 | 68 | 3.33 | 1.23 | 0.51 | 0.26 | 0.44 | 0.31 | 3.05 | 1.66 | 3.01 | 1.88 |
| Example 58 | PTC-58 | 69 | 3.34 | 1.22 | 0.5 | 0.24 | 0.42 | 0.29 | 3.03 | 1.64 | 3.01 | 1.89 |
| Example 59 | PTC-59 | 65 | 3.12 | 1.02 | 0.43 | 0.2 | 0.36 | 0.22 | 2.85 | 1.41 | 2.86 | 1.56 |
| Example 60 | PTC-60 | 67 | 3.36 | 1.21 | 0.51 | 0.22 | 0.42 | 0.3 | 3.08 | 1.65 | 3 | 1.86 |
| Example 61 | PTC-62 | 68 | 2.22 | 0.72 | 0.22 | 0.07 | 0.21 | 0.07 | 2.12 | 0.68 | 1.99 | 0.67 |
| Example 62 | PTC-63 | 69 | 2.21 | 0.73 | 0.21 | 0.08 | 0.22 | 0.08 | 2.14 | 0.7 | 1.98 | 0.68 |
| Example 63 | PTC-65 | 68 | 2.22 | 0.73 | 0.22 | 0.08 | 0.21 | 0.07 | 2.15 | 0.71 | 2.01 | 0.67 |

(continued)

| | Particle number | Percentage biodegradability (%) | Fluffy feel | | | | | | | | | |
|---|---|---|---|---|---|---|---|---|---|---|---|---|
| | | | Particles | | Liquid foundation | | Milky lotion | | Loose powder | | Powder foundation | |
| | | | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) |
| Example 64 | PTC-66 | 69 | 2.23 | 0.71 | 0.21 | 0.07 | 0.22 | 0.07 | 2.14 | 0.69 | 2.01 | 0.68 |
| | | | | | | | | | | | | |
| Comparative Example 1 | PTC-61 | 69 | 0.63 | 0.31 | 0.06 | 0.01 | 0.08 | 0.01 | 1.23 | 0.22 | 0.98 | 0.22 |
| Comparative Example 2 | PTC-64 | 67 | 0.62 | 0.33 | 0.06 | 0.01 | 0.07 | 0.01 | 1.22 | 0.21 | 0.99 | 0.21 |
| Comparative Example 3 | PTC-67 | 67 | 0.99 | 0.43 | 0.09 | 0.02 | 0.11 | 0.02 | 1.56 | 0.38 | 1.45 | 0.38 |
| Comparative Example 4 | PTC-68 | 68 | 0.98 | 0.44 | 0.09 | 0.02 | 0.12 | 0.02 | 1.55 | 0.37 | 1.44 | 0.39 |
| Comparative Example 5 | PTC-69 | 66 | 0.98 | 0.45 | 0.08 | 0.02 | 0.12 | 0.02 | 1.57 | 0.36 | 1.43 | 0.37 |
| Comparative Example 6 | PTC-70 | 67 | 0.97 | 0.44 | 0.09 | 0.02 | 0.11 | 0.02 | 1.56 | 0.38 | 1.4 | 0.38 |
| Comparative Example 7 | PTC-71 | 68 | 0.98 | 0.43 | 0.08 | 0.02 | 0.12 | 0.02 | 1.57 | 0.38 | 1.41 | 0.39 |
| Comparative Example 8 | PTC-72 | 69 | 0.97 | 0.45 | 0.09 | 0.02 | 0.11 | 0.02 | 1.55 | 0.37 | 1.44 | 0.39 |
| | | | | | | | | | | | | |
| Comparative Example 9 | PTC-101 | 79 | 0.63 | 0.31 | 0.06 | 0.01 | 0.08 | 0.01 | 1.24 | 0.22 | 0.98 | 0.19 |
| Comparative Example 10 | PTC-102 | 78 | 0.61 | 0.33 | 0.06 | 0.01 | 0.07 | 0.01 | 1.22 | 0.23 | 0.99 | 0.21 |
| Comparative Example 11 | PTC-103 | 17 | 0.98 | 0.42 | 0.09 | 0.02 | 0.12 | 0.02 | 1.54 | 0.39 | 1.41 | 0.37 |
| Comparative Example 12 | PTC-104 | 25 | 0.99 | 0.44 | 0.09 | 0.02 | 0.11 | 0.02 | 1.53 | 0.38 | 1.42 | 0.38 |

(continued)

| | Particle number | Percentage biodegradability (%) | Fluffy feel | | | | | | | | | |
| --- | --- | --- | --- | --- | --- | --- | --- | --- | --- | --- | --- | --- |
| | | | Particles | | Liquid foundation | | Milky lotion | | Loose powder | | Powder foundation | |
| | | | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) |
| Comparative Example 13 | PT-C-105 | 24 | 0.97 | 0.45 | 0.09 | 0.02 | 0.11 | 0.02 | 1.54 | 0.38 | 1.41 | 0.37 |
| | | | | | | | | | | | | |
| Comparative Example 14 | PTC-111 | 85 | 0.96 | 0.45 | 0.09 | 0.02 | 0.12 | 0.02 | 1.55 | 0.38 | 1.4 | 0.38 |
| Comparative Example 15 | PTC-112 | 88 | 0.97 | 0.43 | 0.09 | 0.02 | 0.11 | 0.02 | 1.54 | 0.37 | 1.42 | 0.37 |

Table 4-4

| | Fluffy feel | | | | | | | | | | | |
|---|---|---|---|---|---|---|---|---|---|---|---|---|
| | Sunscreen cream | | All-in-one gel | | Foundation primer | | Lip primer | | Body powder | | Solid powder eyeshadow | |
| | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) |
| Example 40 | 0.82 | 0.25 | 0.42 | 0.25 | 3.11 | 1.33 | 2.88 | 1.65 | 2.99 | 1.55 | 3.12 | 1.57 |
| Example 41 | 0.82 | 0.24 | 0.41 | 0.24 | 3.12 | 1.32 | 2.87 | 1.64 | 2.98 | 1.51 | 3.13 | 1.55 |
| Example 42 | 0.81 | 0.23 | 0.42 | 0.24 | 3.12 | 1.32 | 2.88 | 1.66 | 2.98 | 1.53 | 3.11 | 1.56 |
| Example 43 | 0.87 | 0.24 | 0.43 | 0.25 | 3.13 | 1.33 | 2.85 | 1.64 | 2.99 | 1.54 | 3.11 | 1.55 |
| Example 44 | 0.8 | 0.24 | 0.42 | 0.26 | 3.12 | 1.32 | 2.82 | 1.63 | 2.98 | 1.53 | 3.09 | 1.56 |
| Example 45 | 0.79 | 0.22 | 0.41 | 0.24 | 3.12 | 1.31 | 2.83 | 1.62 | 2.99 | 1.53 | 3.09 | 1.56 |
| Example 46 | 0.81 | 0.25 | 0.42 | 0.25 | 3.13 | 1.31 | 2.82 | 1.61 | 2.98 | 1.52 | 3.09 | 1.54 |
| Example 47 | 0.66 | 0.19 | 0.35 | 0.21 | 2.88 | 1.11 | 2.66 | 1.44 | 2.77 | 1.24 | 2.88 | 1.37 |
| Example 48 | 0.81 | 0.24 | 0.41 | 0.24 | 3.11 | 1.32 | 2.83 | 1.6 | 2.97 | 1.53 | 3.01 | 1.36 |
| Example 49 | 0.67 | 0.2 | 0.36 | 0.2 | 2.89 | 1.12 | 2.6 | 1.42 | 2.75 | 1.25 | 2.88 | 1.36 |
| Example 50 | 0.79 | 0.25 | 0.41 | 0.25 | 3.11 | 1.33 | 2.82 | 1.61 | 2.97 | 1.54 | 3.11 | 1.56 |
| Example 51 | 0.8 | 0.25 | 0.42 | 0.24 | 3.09 | 1.32 | 2.82 | 1.62 | 2.97 | 1.54 | 3.09 | 1.55 |
| Example 52 | 0.82 | 0.25 | 0.41 | 0.25 | 3.1 | 1.31 | 2.84 | 1.61 | 2.97 | 1.54 | 3.08 | 1.55 |
| Example 53 | 0.81 | 0.24 | 0.42 | 0.24 | 3.08 | 1.33 | 2.83 | 1.6 | 2.96 | 1.55 | 3.1 | 1.54 |
| Example 54 | 0.8 | 0.23 | 0.41 | 0.25 | 3.09 | 1.32 | 2.82 | 1.62 | 2.96 | 1.56 | 3.11 | 1.54 |
| Example 55 | 0.79 | 0.24 | 0.42 | 0.26 | 3.07 | 1.35 | 2.81 | 1.63 | 2.98 | 1.54 | 3.1 | 1.55 |
| Example 56 | 0.8 | 0.23 | 0.42 | 0.24 | 3.07 | 1.32 | 2.82 | 1.64 | 2.97 | 1.55 | 3.09 | 1.56 |
| Example 57 | 0.99 | 0.29 | 0.46 | 0.29 | 3.25 | 1.49 | 299 | 1.8 | 3.12 | 1.71 | 3.31 | 1.71 |
| Example 58 | 0.98 | 0.28 | 0.46 | 0.28 | 3.28 | 1.48 | 3 | 1.8 | 3.12 | 1.7 | 3.29 | 1.72 |
| Example 59 | 0.81 | 0.23 | 0.43 | 0.25 | 3.06 | 1.33 | 2.81 | 1.63 | 2.96 | 1.55 | 3.09 | 1.55 |
| Example 60 | 0.97 | 0.29 | 0.47 | 0.28 | 3.27 | 1.48 | 2.99 | 1.79 | 3.09 | 1.7 | 3.29 | 1.71 |
| Example 61 | 0.34 | 0.09 | 0.22 | 0.07 | 212 | 0.69 | 2.01 | 0.68 | 2.25 | 0.69 | 2.41 | 0.78 |
| Example 62 | 0.35 | 0.09 | 0.21 | 0.08 | 2.11 | 0.71 | 2 | 0.69 | 2.22 | 0.68 | 2.39 | 0.77 |
| Example 63 | 0.36 | 0.09 | 0.21 | 0.08 | 213 | 0.69 | 1.99 | 0.68 | 2.21 | 0.67 | 2.42 | 0.78 |

(continued)

| | Fluffy feel | | | | | | | | | | | |
|---|---|---|---|---|---|---|---|---|---|---|---|---|
| | Sunscreen cream | | All-in-one gel | | Foundation primer | | Lip primer | | Body powder | | Solid powder eyeshadow | |
| | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) |
| Example 64 | 0.35 | 0.09 | 0.23 | 0.07 | 212 | 0.7 | 1.98 | 0.69 | 2.25 | 0.68 | 2.39 | 0.79 |
| | | | | | | | | | | | | |
| Comparative Example 1 | 0.09 | 0.01 | 0.08 | 0.01 | 1.21 | 0.21 | 1.11 | 0.25 | 1.02 | 0.21 | 1.33 | 0.16 |
| Comparative Example 2 | 0.09 | 0.01 | 0.07 | 0.01 | 1.19 | 0.22 | 1.12 | 0.25 | 1.04 | 0.22 | 1.32 | 0.17 |
| Comparative Example 3 | 0.19 | 0.04 | 0.12 | 0.02 | 1.55 | 0.39 | 1.55 | 0.31 | 1.34 | 0.31 | 1.52 | 0.28 |
| Comparative Example 4 | 0.19 | 0.04 | 0.11 | 0.02 | 1.56 | 0.38 | 1.51 | 0.33 | 1.33 | 0.31 | 1.51 | 0.27 |
| Comparative Example 5 | 0.18 | 0.04 | 0.12 | 0.02 | 1.55 | 0.38 | 1.5 | 0.31 | 1.32 | 0.32 | 1.51 | 0.27 |
| Comparative Example 6 | 0.19 | 0.04 | 0.11 | 0.02 | 1.57 | 0.39 | 1.53 | 0.32 | 1.33 | 0.31 | 1.51 | 0.25 |
| Comparative Example 7 | 0.19 | 0.04 | 0.11 | 0.02 | 1.58 | 0.38 | 1.55 | 0.31 | 1.34 | 0.31 | 1.52 | 0.24 |
| Comparative Example 8 | 0.18 | 0.03 | 0.11 | 0.02 | 1.56 | 0.37 | 1.5 | 0.33 | 1.32 | 0.31 | 1.51 | 0.24 |
| | | | | | | | | | | | | |
| Comparative Example 9 | 0.09 | 0.01 | 0.08 | 0.01 | 1.19 | 0.2 | 1.12 | 0.28 | 1.04 | 0.21 | 1.32 | 0.17 |
| Comparative Example 10 | 0.09 | 0.01 | 0.07 | 0.01 | 1.19 | 0.21 | 1.13 | 0.26 | 1.03 | 0.22 | 1.33 | 1.16 |
| Comparative Example 11 | 0.19 | 0.04 | 0.12 | 0.02 | 1.56 | 0.37 | 1.53 | 0.3 | 1.32 | 0.31 | 1.5 | 0.24 |
| Comparative Example 12 | 0.18 | 0.03 | 0.12 | 0.02 | 1.55 | 0.37 | 1.54 | 0.31 | 1.33 | 0.32 | 1.53 | 0.25 |

(continued)

| | Fluffy feel | | | | | | | | | | | |
| --- | --- | --- | --- | --- | --- | --- | --- | --- | --- | --- | --- | --- |
| | Sunscreen cream | | All-in-one gel | | Foundation primer | | Lip primer | | Body powder | | Solid powder eyeshadow | |
| | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) | Compression energy (J) | Work of recovery (W) |
| Comparative Example 13 | 0.19 | 0.03 | 0.11 | 0.02 | 1.56 | 0.36 | 1.5 | 0.3 | 1.31 | 0.31 | 1.52 | 0.22 |
| | | | | | | | | | | | | |
| Comparative Example 14 | 0.19 | 0.04 | 0.11 | 0.02 | 1.57 | 0.37 | 1.51 | 0.32 | 1.32 | 0.31 | 1.51 | 0.21 |
| Comparative Example 15 | 0.19 | 0.04 | 0.11 | 0.02 | 1.57 | 0.37 | 1.51 | 0.31 | 1.32 | 0.31 | 1.52 | 0.23 |

From these results, it can be seen that the cellulosic particles of the Examples have a fluffy feel.

The foregoing description of the exemplary embodiments of the present disclosure has been provided for the purposes of illustration and description. It is not intended to be exhaustive or to limit the disclosure to the precise forms disclosed. Obviously, many modifications and variations will be apparent to practitioners skilled in the art. The embodiments were chosen and described in order to best explain the principles of the disclosure and its practical applications, thereby enabling others skilled in the art to understand the disclosure for various embodiments and with the various modifications as are suited to the particular use contemplated. It is intended that the scope of the disclosure be defined by the following claims.

**Claims**

1. A cellulosic particle comprising:

   cellulose as a base constituent; and
   at least one selected from the group consisting of a hydrocarbon ester and a ketone, wherein:

   the hydrocarbon ester is at least one selected from the group consisting of ethyl acetate, butyl acetate, and ethyl propionate,
   the ketone is at least one selected from the group consisting of methyl ethyl ketone, methyl isobutyl ketone, and acetone,
   at least one of an amount of the hydrocarbon ester and an amount of the ketone is 3 ppm or more and 1000 ppm or less relative to the cellulosic particles, ppm being by mass, and the amount of the hydrocarbon ester and the amount of the ketone are measured in accordance with
   the method outlined the description, and an amount of the cellulose relative to the cellulosic particle is 90% by mass or more.

2. The cellulosic particle according to claim 1, wherein:
   the cellulosic particle has:

   a core particle with a cellulose content relative to the core particle of 90% by mass or more; and
   a coating layer covering the core particle and containing at least one selected from the group consisting of a fatty acid, a fatty acid metallic salt, and an amino acid compound.

3. The cellulosic particle according to claim 2, wherein:

   the fatty acid is a fatty acid having 16 or more and 22 or fewer carbon atoms; and
   a number of carbon atoms in the fatty acid metallic salt is 16 or more and 22 or fewer.

4. The cellulosic particle according to claim 2 or 3, wherein:

   the fatty acid is a saturated fatty acid; and
   the fatty acid metallic salt is a saturated fatty acid metallic salt.

5. The cellulosic particle according to any one of claims 2 to 4, wherein:

   the cellulosic particle has an intermediate layer between the core particle and the coating layer; and
   the intermediate layer is at least one selected from the group consisting of a polyamine compound, a polyquaternium, a polysaccharide compound, and a polyacrylic acid.

6. The cellulosic particle according to any one of claims 1 to 5, wherein:
   the cellulosic particle has an inorganic particle as an external additive.

7. The cellulosic particle according to any one of claims 1 to 6, wherein:
   a volume-average particle diameter of the cellulosic particles is 3 $\mu$m or more and less than 10 $\mu$m and measured in accordance with the method outlined in the description.

8. The cellulosic particle according to any one of claims 1 to 7, wherein:

an upper geometric standard deviation by number GSDv of the cellulosic particles is 1.0 or greater and 1.7 or less and measured in accordance with the method outlined in the description.

9. The cellulosic particle according to any one of claims 1 to 8, wherein:
   sphericity of the cellulosic particle is 0.7 or greater and measured in accordance with the method outlined in the description.

10. The cellulosic particle according to any one of claims 1 to 9, wherein:
    surface smoothness of the cellulosic particle is 50% or more and measured in accordance with the method outlined in the description.

11. The cellulosic particle according to any one of claims 1 to 10, wherein:
    a number-average molecular weight of the cellulose is 37000 or more and measured by gel permeation chromatography.

12. The cellulosic particle according to claim 11, wherein:
    the number-average molecular weight of the cellulose is 45000 or more.

**Patentansprüche**

1. Zellulosepartikel, umfassend:

   Zellulose als einen Basisbestandteil; und
   mindestens einen, der aus der Gruppe, die aus einem Kohlenwasserstoffester und einem Keton besteht, ausgewählt wird, wobei:

   der Kohlenwasserstoffester mindestens einer ist, der aus der Gruppe, die aus Ethylacetat, Butylacetat und Ethylpropionat besteht, ausgewählt wird,
   das Keton mindestens einer ist, der aus der Gruppe, die aus Methyläthylketon, Methylisobutylketon und Aceton besteht, ausgewählt wird,
   mindestens eine von einer Menge des Kohlenwasserstoffesters und einer Menge des Ketons 3 ppm oder mehr und 1000 ppm oder weniger relativ zu den Zellulosepartikeln beträgt, wobei ppm auf Massenbasis sind und die Menge des Kohlenwasserstoffesters und die Menge des Ketons in Übereinstimmung mit dem in der Beschreibung skizzierten Verfahren gemessen werden, und
   wobei eine Menge der Zellulose relativ zu den Zellulosepartikeln 90 Massen-% oder mehr beträgt,

2. Zellulosepartikel nach Anspruch 1, wobei:
   das Zellulosepartikel aufweist:

   ein Kernpartikel mit einem Zellulosegehalt relativ zu dem Kernpartikel von 90 Massen-% oder mehr; und
   eine Beschichtungsschicht, die das Kernpartikel abdeckt und mindestens eine, die aus der Gruppe ausgewählt wird, die aus einer Fettsäure, einem Fettsäure-Metallsalz und einer Aminosäureverbindung besteht, enthält; und

3. Zellulosepartikel nach Anspruch 2, wobei:

   die Fettsäure eine Fettsäure ist, die 16 oder mehr und 22 oder weniger Kohlenstoffatomen aufweist; und
   eine Anzahl an Kohlenstoffatomen in dem Fettsäure-Metallsalz 16 oder mehr und 22 oder weniger beträgt.

4. Zellulosepartikel nach Anspruch 2 oder 3, wobei:

   die Fettsäure eine gesättigte Fettsäure ist; und
   das Fettsäure-Metallsalz ein gesättigtes Fettsäure-Metallsalz ist.

5. Zellulosepartikel nach einem der Ansprüche 2 bis 4, wobei:

   das Zellulosepartikel eine Zwischenschicht zwischen dem Kernpartikel und der Beschichtungsschicht aufweist;

und

die Zwischenschicht mindestens eine ist, die aus der Gruppe, die aus einer Polyaminverbindung, einem Polyquaternium, einer Polysaccharidverbindung und einer Polyacrylsäure besteht, ausgewählt wird.

**6.** Zellulosepartikel nach einem der Ansprüche 1 bis 5, wobei:
das Zellulosepartikel ein anorganisches Partikel als ein externes Additiv aufweist.

**7.** Zellulosepartikel nach einem der Ansprüche 1 bis 6, wobei:
ein volumendurchschnittlicher Partikeldurchmesser der Zellulosepartikel 3 µm oder mehr und weniger als 10 µm beträgt und in Übereinstimmung mit dem in der Beschreibung skizzierten Verfahren gemessen wird.

**8.** Zellulosepartikel nach einem der Ansprüche 1 bis 7, wobei:
eine obere geometrische Standardabweichung GSDv nach Anzahl der Zellulosepartikel 1,0 oder größer und 1,7 oder kleiner ist und in Übereinstimmung mit dem in der Beschreibung skizzierten Verfahren gemessen wird.

**9.** Zellulosepartikel nach einem der Ansprüche 1 bis 8, wobei:
Kugeligkeit des Zellulosepartikels 0,7 oder größer ist und in Übereinstimmung mit dem in der Beschreibung skizzierten Verfahren gemessen wird.

**10.** Zellulosepartikel nach einem der Ansprüche 1 bis 9, wobei:
Oberflächenglätte des Zellulosepartikels 50 % oder mehr beträgt und in Übereinstimmung mit dem in der Beschreibung skizzierten Verfahren gemessen wird.

**11.** Zellulosepartikel nach einem der Ansprüche 1 bis 10, wobei:
ein zahlenmittleres Molekulargewicht der Zellulose 37000 oder mehr beträgt und durch Gelpermeationschromatographie gemessen wird.

**12.** Zellulosepartikel nach Anspruch 11, wobei:
das zahlenmittlere Molekulargewicht der Zellulose 45000 oder mehr beträgt.


**Revendications**

**1.** Particule cellulosique comprenant :

de la cellulose en tant que constituent de base ; et
au moins un choisi dans le groupe constitué d'un ester d'hydrocarbure et d'une cétone,
dans laquelle :

l'ester d'hydrocarbure est au moins un choisi dans le groupe constitué d'acétate d'éthyle, d'acétate de butyle et de propionate d'éthyle,
la cétone est au moins un choisi dans le groupe constitué de méthyléthylcétone, de méthylisobutylcétone et d'acétone,
au moins l'une d'une quantité de l'ester d'hydrocarbure et d'une quantité de la cétone est de 3 ppm ou plus et de 1000 ppm ou moins par rapport aux particules cellulosiques,
les ppm étant en masse, et la quantité de l'ester d'hydrocarbure et la quantité de la cétone sont mesurées conformément à la méthode décrite dans la description, et
une quantité de la cellulose par rapport à la particule cellulosique est de 90 % en masse ou plus.

**2.** Particule cellulosique selon la revendication 1, dans laquelle :
la particule cellulosique comporte :

une particule cœur ayant une teneur en cellulose par rapport à la particule cœur de 90 % en masse ou plus ; et
une couche de revêtement recouvrant la particule cœur et contenant au moins un choisi dans le groupe constitué d'un acide gras, d'un sel métallique d'acide gras et d'un composé d'acide aminé ; et

**3.** Particule cellulosique selon la revendication 2, dans laquelle :

EP 4 410 383 B1

l'acide gras est un acide gras ayant 16 atomes de carbone ou plus et 22 atomes de carbone ou moins ; et
un nombre d'atomes de carbone dans le sel métallique d'acide gras est de 16 ou plus et de 22 ou moins.

4. Particule cellulosique selon la revendication 2 ou la revendication 3, dans laquelle :

   l'acide gras est un acide gras saturé ; et
   le sel métallique d'acide gras est un sel métallique d'acide gras saturé.

5. Particule cellulosique selon l'une quelconque des revendications 2 à **4,** dans laquelle :

   la particule cellulosique comporte une couche intermédiaire entre la particule cœur et la couche de revêtement ;
   et
   la couche intermédiaire est au moins un choisi dans le groupe constitué d'un composé de polyamine, d'un
   polyquaternium, d'un composé de polysaccharide et d'un acide polyacrylique.

6. Particule cellulosique selon l'une quelconque des revendications 1 à 5, dans laquelle :
   la particule cellulosique comporte une particule inorganique en tant qu'additif externe.

7. Particule cellulosique selon l'une quelconque des revendications 1 à 6, dans laquelle :
   un diamètre moyen en volume des particules cellulosiques est de 3 $\mu$m ou plus et inférieur à 10 $\mu$m et est mesuré
   conformément à la méthode décrite dans la description.

8. Particule cellulosique selon l'une quelconque des revendications 1 à 7, dans laquelle :
   un écart type géométrique supérieur en nombre GSDv des particules cellulosiques est de 1,0 ou plus et de 1,7 ou
   moins et est mesuré conformément à la méthode décrite dans la description.

9. Particule cellulosique selon l'une quelconque des revendications 1 à 8, dans laquelle :
   la sphéricité de la particule cellulosique est de 0,7 ou plus et est mesurée conformément à la méthode décrite dans la
   description.

10. Particule cellulosique selon l'une quelconque des revendications 1 à 9, dans laquelle :
    la rugosité de surface de la particule cellulosique est de 50 % ou plus et est mesurée conformément à la méthode
    décrite dans la description.

11. Particule cellulosique selon l'une quelconque des revendications 1 à 10, dans laquelle :
    un poids moléculaire moyen en nombre de la cellulose est de 37 000 ou plus et est mesuré par chromatographie par
    perméation de gel.

12. Particule cellulosique selon la revendication 11, dans laquelle :
    le poids moléculaire moyen en nombre de la cellulose est de 45 000 ou plus.

**REFERENCES CITED IN THE DESCRIPTION**

**Patent documents cited in the description**

- JP 2022099605 A
- JP 2020132616 A
- WO 2022137679 A1

- EP 4116357 A1
- CN 114555678 A
- JP 6921293 B